# FASCICULE DE BREVET EUROPEEN

(11) **EP 0 828 742 B1**
(45) Date de publication et mention de la délivrance du brevet: **19.12.2001**
(21) Numéro de dépôt: 97919432.1
(22) Date de dépôt: 27.03.1997
(51) Int. Cl.: C07D 487/06, A61K 31/55, C07D 519/00

(54) **DIAZEPINO-INDOLES INHIBITEURS DE PHOSPHODIESTERASES 4**
DIAZEPINO-INDOLE ALS PHOSPHODIESTERASE IV INHIBITOREN
DIAZEPINO-INDOLES AS PHOSPHODIESTERASE IV INHIBITORS

(30) Priorité: 29.03.1996 FR 9604013
(43) Date de publication de la demande: 18.03.1998
(60) Demande divisionnaire: 01114421.9
(73) Titulaire: INSTITUT DE RECHERCHE JOUVEINAL (I.R.J), 94265 Fresnes Cedex (FR)
(72) Inventeur: PASCAL, Yves, F-92500 Rueil-Malmaison (FR); JACOBELLI, Henry, F-91550 Paray-Vieille-Poste (FR); CALVET, Alain, Ann Arbor, MI 48104 (US); PAYNE, Adrian, F-92160 Antony (FR); DAHL, Svein, G., F-91190 Gif-sur-Yvette (FR)
(74) Mandataire: Dufresne, Guillaume Alain François
(86) Numéro de dépôt international: FR9700557
(87) Numéro de publication internationale: WO9736905

(56) Documents cités:
- EP-A- 0 265 734
- WO-A-96/11690

## Description

### Domaine de l'invention

La présente invention est relative à de nouveaux [1,4]diazépino[6,7,1-*hi*]indoles utiles pour la préparation de médicaments permettant de traiter des affections relevant d'une thérapie par un inhibiteur de phosphodiestérases 4. Ces médicaments sont utiles notamment comme anti-inflammatoires, anti-allergiques, bronchodilatateurs, ou anti-asthmatiques, et sont dénués d'effets secondaires digestifs ou cardiaques.

### Arrière-plan technologique de l'invention

Différemment des propriétés révélées par la présente invention, l'art antérieur fait état de [1,4]diazépino[6,7,1-*hi*]indoles pour lesquels il est décrit des propriétés antagonistes de la cholécystokinine (CCK) et/ou de la gastrine, et qui sont proposés pour des affections du tube digestif : estomac, intestin, pancréas et vésicule biliaire, et notamment les troubles de la satiété.

Ainsi, la demande de brevet européen n° 340 064 décrit des composés de formule : 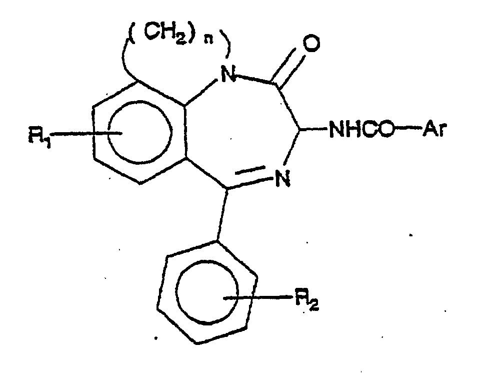 dans laquelle R₁ et R₂ sont hydrogène ou halogène, Ar est indolyle ou phényle et n est 2 ou 3. Ces composés sont des antagonistes périphériques de la cholécystokinine (CCK_{A}).

La demande de brevet européen n° 360 079 décrit des composés antagonistes périphériques et/ou centraux de la CCK de formule : 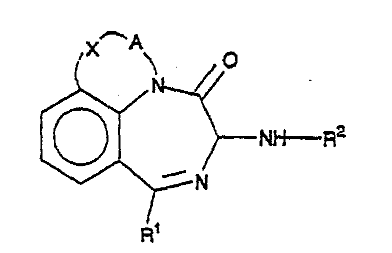 dans laquelle R¹ est aryle éventuellement substitué, X est oxygène ou méthylène éventuellement substitué par un radical alkyle inférieur, A est une liaison ou alkylène inférieur qui peut avoir un ou des groupes alkyles inférieurs, R² est hydrogène, ou acyle.

La demande de brevet français n° FR 94 12282 non publiée (WO-A-96/11690) décrit l'application de dérivés de diazépinoindoles de formule 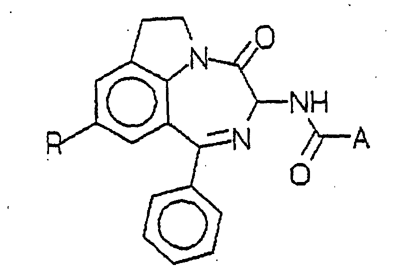 dans laquelle R est hydrogène, alkyle inférieur, alkoxy inférieur, A est un noyau aromatique éventuellement substitué, dont certains sont nouveaux, pour la préparation de médicaments destinés au traitement d'affections relevant d'un inhibiteur de phosphodiestérases 4.

En ce qui concerne l'inhibition des phosphodiestérases, il est rappelé que l'adénosine 3', 5'-monophosphate cyclique (AMPc) est un second messager intracellulaire ubiquitaire, intermédiaire entre un premier messager (hormone, neurotransmetteur, ou autacoïde) et les réponses fonctionnelles cellulaires : le premier messager stimule l'enzyme responsable de la synthèse de l'AMPc ; l'AMPc intervient alors, selon les cellules en cause, dans de très nombreuses fonctions : métaboliques, contractiles, ou sécrétoires.

- Les effets de l'AMPc prennent fin lorsqu'il est dégradé par les phosphodiestérases des nucléotides cycliques, enzymes intracellulaires qui catalysent son hydrolyse en adénosine 5'-monophosphate inactive.

On distingue chez les mammifères au moins cinq grandes familles de phosphodiestérases des nucléotides cycliques (PDE) numérotées de 1 à 5 selon leur structure, leur comportement cinétique, leur spécificité de substrat, ou leur sensibilité à des effecteurs (Beavo J.A. *et al*. (1990) Trends Pharmacol. Sci. 11, 150-155. Beavo J.A. *et al*. (1994) Molecular Pharmacol. 46, 399-405). Les PDE4 sont spécifiques de l'AMPc.

Des composés inhibiteurs non spécifiques de phosphodiestérases sont connus, qui inhibent plusieurs familles d'enzymes. C'est le cas de certaines meiylxanthines comme la théophylline. Ces composés ont un index thérapeutique faible, notamment en raison de leur action sur des types de PDE présents dans des cellules autres que les cellules cibles. A l'inverse, certaines familles de PDE peuvent être inhibées sélectivement par divers agents pharmacologiques : l'hydrolyse des nucléotides cycliques est ralentie et donc leur concentration augmente dans les seules cellules où se trouve le type de PDE sensible à l'inhibiteur.

Un intérêt particulier se manifeste pour les phosphodiestérases 4 (PDE4), qui ont été identifiées dans de nombreux tissus dont le système nerveux.central, le coeur, l'endothélium vasculaire, le muscle lisse vasculaire et celui des voies aériennes, les lignées myéloïdes et lymphoïdes.

Une augmentation de l'AMPc dans les cellules impliquées dans l'inflammation inhibe leur activation : inhibition de la synthèse et de la libération de médiateurs au niveau des mastocytes, des monocytes, des polynucléaires éosinophiles et basophiles, inhibition du chimiotactisme et de la dégranulation des polynucléaires neutrophiles et éosinophiles, inhibition des divisions et de la différenciation des lymphocytes.

Les cytokines, notamment TNF et interleukines, produits par différents types de leukocytes comme les lymphocytes T et les polynucléaires éosinophiles, jouent un rôle important dans le déclenchement des manifestations inflammatoires en particulier en réponse à une stimulation par un allergène au niveau des voies respiratoires.

D'autre part, l'AMPc diminue le tonus des fibres musculaires lisses des voies aériennes ; les inhibiteurs de PDE4 déterminent une bronchorelaxation.

On peut donc s'attendre à ce que des inhibiteurs sélectifs de PDE4 possèdent une activité thérapeutique comme médicaments anti-inflammatoires, anti-allergiques, bronchodilatateurs, et dans le traitement de l'asthme, où l'on observe une infiltration des voies aériennes par des cellules inflammatoires et une bronchoconstriction.

La théophylline est très largement utilisée depuis longtemps dans le traitement de l'asthme, et bien que son mécanisme d'action soit complexe, l'inhibition de PDE contribue à son action, mais aussi à certains effets indésirables comme les nausées et les céphalées.

Depuis quelques années une recherche extensive a été menée pour l'obtention et la mise au point d'inhibiteurs puissants de PDE4. Elle s'avère difficile du fait que beaucoup des inhibiteurs potentiels de PDE4 ne sont pas dénués d'activité sur les phosphodiestérases des autres familles.

A ce jour, le manque de sélectivité des inhibiteurs de PDE4 représente donc un problème important, étant donné l'étendue des fonctions régulées par l'AMPc, ledit problème devant être considéré encore mal ou non résolu. Il existe donc un besoin pour des inhibiteurs puissants et sélectifs de PDE4, c'est-à-dire n'ayant pas d'action vis-à-vis des PDE appartenant à d'autres familles.

Ainsi le rolipram (DCI), dérivé de pyrrolidone synthétisé dès 1975, est considéré comme représentatif des inhibiteurs spécifiques de PDE4. De nombreux composés apparentés au rolipram ont été synthétisés en vue de leur utilisation comme inhibiteurs de PDE4. In vitro, le rolipram inhibe l'activité des cellules inflammatoires chez les rongeurs : inhibition de la synthèse des médiateurs par les mastocytes, les polynucléaires éosinophiles et basophiles, et les monocytes ; inhibition du chimiotactisme et de la dégranulation des polynucléaires.

Le rolipram a été proposé comme antidépresseur ; cependant son utilisation s'accompagne d'effets indésirables à type de nausées et de vomissements.

### Sommaire de l'invention

Or, surmontant les difficuités rapportées dans l'état de la technique, on a maintenant trouvé de nouveaux dérivés de [1,4]diazépino[6,7,1-*hi*]indoles, qui sont de puissants inhibiteurs de PDE4 à des concentrations auxquelles ils ont peu ou pas d'action sur les autres familles de PDE.

L'invention concerne les diazépino-indoles de formule (I) 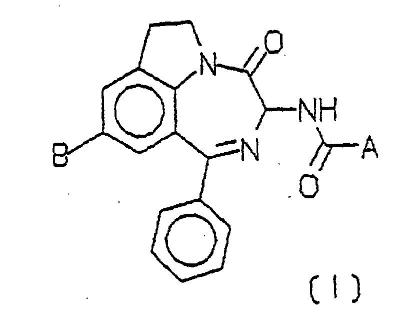 dans laquelle :
- A est aryle, hétéroaryle azoté, chacun éventuellement substitué par un à trois groupes indépendamment choisis parmi halogène, alkyle inférieur, haloalkyle inférieur, alkoxy inférieur, cycloalkyloxy, amino, alkyloxycarbonylamino ou alkylcarbonylamino inférieur ;
- B est un radical hydroxy ou amino, lui-même éventuellement substitué, leur procédé de préparation et leur application à l'obtention de médicaments destinés à traiter les affections relevant d'une thérapie par l'inhibition de PDE4.

### Description détaillée de l'invention

L'invention vise les diazépino-indoles de formule (I) 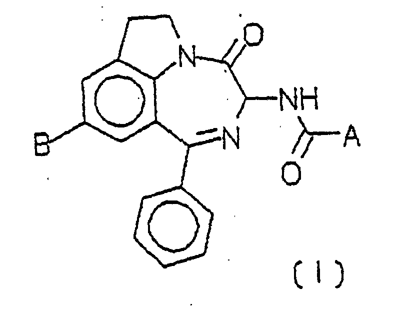 dans laquelle :
- A est aryle, hétéroaryle azoté, chacun éventuellement substitué par un à trois groupes indépendamment choisis parmi halogène, alkyle inférieur, haloalkyle inférieur, alkoxy inférieur, cycloalkyloxy, amino, alkyloxycarbonylamino ou alkylcarbonylamino inférieur ;
- B est :
   1°) -OR₁, R₁ étant -H ou R₄,
   2°) -NR₂R₃, R₂ étant -C(NH)NH₂, et R₃ étant -H,
   3°) -NR₂R₃, R₂ étant R₄, et R₃ étant -H ,
   4°) -NR₂R₃, R₂ et R₃ étant indépendamment -H ou alkyle inférieur , ou
   5°) -N-R₂-R₃, R₂ et R₃ formant, ensemble avec l'atome azote auquel ils sont reliés, un hétérocycle saturé de cinq à sept chaînons pouvant comprendre, comme second hétéroatome non relié directement à l'atome d'azote, un oxygène, un soufre, ou un azote;
- R₄ est :
   1°) -CH₂-CO₂H,
   2°) -CO-(CH₂)ₚ-CO₂H,
   3°) -CO-A, où A est à la définition indiquée ci-dessus,
   4°) -CO-CH = CH-CO₂H,
   5°) -CO-(CH₂)ₙ-CH₃, n étant un entier égal ou supérieur à 0 et inférieur ou égal à 18,
   6°) -CO-(CH₂-O-CH₂)ₚ-CH₂-O-CH₃,
   7°) -CO-(CH₂-O-CH₂)ₚ-CO₂H,
   8°) -(CH₂)ₚ-NR₅R₆, R₅ et R₆ étant indépendamment -H ou alkyle inférieur, ou
   9°) -(CH₂)ₚ-N-R₅-R₆, R₅ et R₆ formant, ensemble avec l'atome d'azote auquel ils sont reliés, un hétérocycle saturé de cinq à sept chaînons pouvant comprendre, comme second hétéroatome non relié directement à l'atome d'azote, un oxygène, un soufre, ou un azote ;
- p est un entier égal à 2, 3, ou 4 ;
leurs formes racémiques et leurs isomères notamment ceux de configuration déterminée par le carbone 3 du noyau diazépino-indol-4-one,
ainsi que leurs sels pharmaceutiquement acceptables.

Dans ce qui précède ainsi que dans ce qui suit :
- par aryle on entend phényle ou naphtyle ;
- par hétéroaryle azoté on entend un monocycle ou un polycycle non saturé contenant au moins un atome d'azote et, de préférence, des hétéromonocycles ayant de cinq à sept sommets et contenant de 1 à 4 atomes d'azote, ou bien des hétérocycles condensés non saturés contenant de 1 à 4 atomes d'azote, éventuellement méthylés ou éthylés sur un atome d'azote chargé positivement ;
- par halogène on entend le fluor, le chlore, le brome ou l'iode ;
- par inférieur, en ce qui concerne les radicaux comprenant une séquence alkyle, on entend qu' alkyle soit linéaire ou ramifié et comporte de un à quatre atomes de carbone, ou encore représente le radical cyclopropylméthyle ;
- par cycloalkyle on entend les groupes cyclopropyles, cyclobutyles, cyclopentyles et cyclohexyles ;
- par haloalkyle on entend un mono-, di-, ou trihaloalkyle.

On trouvera une revue générale des sels acceptables en pharmacie dans J. Pharm. Sci., 1977, 66, 1-19. Toutefois, par sel pharmacologiquement acceptable d'un composé de formule (I) présentant une portion basique on entend les sels d'addition des composés de formule (I) que l'on forme à partir d'acides minéraux ou organiques non toxiques comme par exemple les sels d'acides bromhydrique, chlorhydrique, sulfurique, phosphorique, nitrique, acétique, succinique, tartrique, citrique, maléique, hydroxymaléique, benzoïque, fumarique, toluène-sulfonique, isethionique et autres. Les divers sels d'ammonium quaternaires des dérivés (I) sont également inclus dans cette catégorie des composés de l'invention. Et par sel pharmacologiquement acceptable d'un composé de formule (I) présentant une portion acide on entend les sels usuels des composés de formule (I) que l'on forme à partir de bases minérales ou organiques non toxiques comme par exemple les hydroxydes des métaux alcalins et alcalino-terreux (sodium, potassium, magnésium et calcium), les amines (dibenzyléthylènediamine, triméthylamine, pipéridine, pyrrolidine, benzylamine et autres) ou encore les hydroxydes d'ammoniums quaternaires comme l'hydroxyde de tétraméthylammonium.

On préfère de manière générale les diazépino-indoles de formule (I) dans laquelle l'atome de carbone asymétrique en position alpha par rapport au carbonyle "3-one" du cycle diazépine possède la configuration absolue (R) selon la règle de Cahn-Ingold-Prelog.

Un groupe de composés (I) dans lesquels B est OR₁ ou NR₂R₃ avec R₁, R₂, R₃ représentant l'hydrogène est préféré.

Un autre ensemble de produits (I) constitué par ceux dans lesquels A est aryle substitué par 1 à 3 groupes indépendamment choisis parmi halogène, amino, alkoxy ou alkyloxycarbonylamino inférieur est avantageusement préféré de même que l'ensemble de produits (I) dans lesquels A est hétéroaryle monocyclique comprenant de 1 à 2 atomes d'azote ou bicyclique comprenant de 1 à 4 atomes d'azote.

Plus, particulièrement on préfère les composés (I) suivants :
- le (3R)Isoquinoline-3-acide carboxylique (9-hydroxy-4-oxo-1-phényl-3,4,6,7-tétrahydro-[1,4]diazépino[6,7,1-hi]indol-3-yl)-amide
- le (3R)4-t-butyloxycarbonylamino-N-(9-amino-4-oxo-1-phényl-3,4,6,7-tétrahydro-[1,4]diazépino[6,7,1-hi]indol-3-yl)-benzamide
- le (3R)4-amino-N-(9-amino-4-oxo-1-phényl-3,4,6,7-tétrahydro-[1,4]diazépino[6,7,1-hi]indol-3-yl)-3,5-dichloro-benzamide
- le (3R)4-amino-N-(9-amino-4-oxo-1-phényl-3,4,6,7-tétrahydro-[1,4]diazépino[6,7,1-hi]indol-3-yl)-5-chloro-2-méthoxy-benzamide
- le (3R)N-(9-amino-4-oxo-1-phényl-3,4,6,7-tétrahydro-[1,4]diazépino[6,7,1-hi]indol -3-yl)-isonicotinamide
- le (3R)3-t.butyloxycarbonylamino-N-(9-amino-4-oxo-1-phényl-3,4,6,7-tétrahydro-[1,4]diazépino[6,7,1-hi] indol-3-yl)-isonicotinamide et son sel d'addition avec l'acide sulfurique
- le (3R)isoquinoline-3-acide carboxylique (9-amino-4-oxo-1-phényl-3,4,6,7-tétrahydro-[1,4]diazépino[6,7,1-hi]indol-3-yl)-amide
- le (3R)quinoline-3-acide carboxylique (9-amino-4-oxo-1-phényl-3,4,6,7-tétrahydro-[1,4]diazépino[6,7,1-hi]indol-3-yl)-amide
- le (3R)4,7-diméthyl-pyrazolo[5,1-c][1,2,4]triazine-3-acide carboxylique (9-amino-4-oxo-1-phényl-3,4,6,7-tétrahydro-[1,4]diazépino[6,7,1-hi]indol-3-yl)-amide
- le (3R)4-amino-3,5-dichloro-N-(9-diméthylamino-4-oxo-1-phényl-3,4,6,7-tétrahydro-[1,4]diazépino[6,7,1-hi]indol-3-yl)-benzamide.
- le (3R)N-(9-amino-4-oxo-1-phényl-3,4,6,7-tétrahydro-[1,4]diazépino[6,7,1-hi]indol-3-yl)-2-benzofuranecarboxaimde,
- le(3R)4,7-diméthyl-pyrazolo[5,1-c][1,2,4]triazine-3-carboxylique acide [4-oxo-1-phényl-9-(pyrrolidin-1-yl)-3,4,6,7-tétrahydro-[1,4]diazépino[6,7,1-hi]indol-3-yl]-amide,

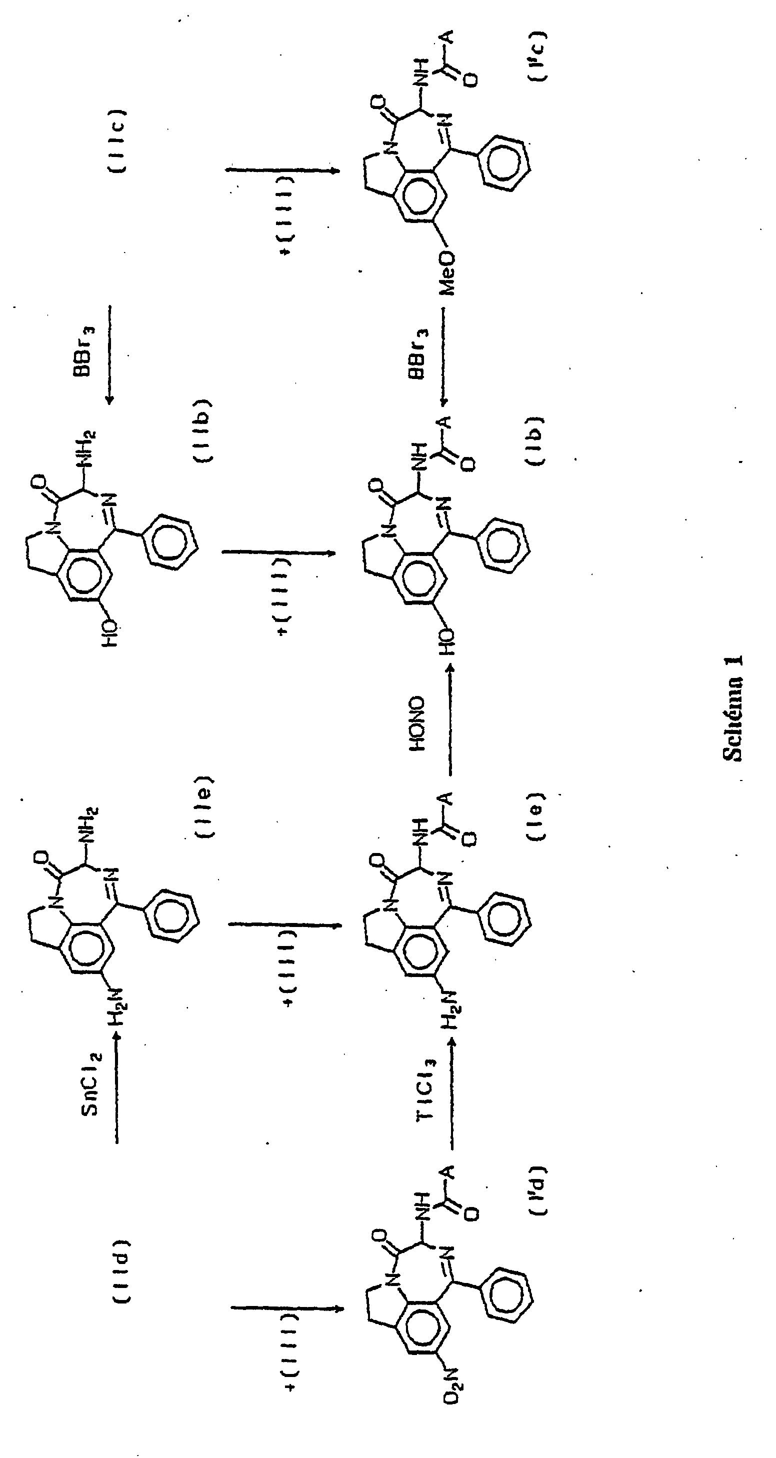

Un autre aspect de l'invention vise un procédé de préparation des diazépino-indoles (I) qui consiste, tel que montré au schéma 1 :
**a)** pour obtenir les composés (I_{b}), de formule (I) dans laquelle B est un groupe -OH :
   - à acyler un amino-diazépino-indole intermédiaire (II_{b}), de formule (II) dans laquelle B est un groupe -OH, par un réactif (III), de formule Z-CO-A dans laquelle A est tel que défini pour (I) et Z représente un halogène, un groupe hydroxy, un groupe azido, un groupe imidazol-1-yle ou un groupe -O-CO-Z₁, Z₁ pouvant être, outre A, un radical alkyle ou alkoxy encombré comportant de 3 à 6 atomes de carbone, ou bien encore Z peut être un groupe O-Z₂, Z₂ étant un groupe aromatique comportant un ou deux cycles substitués par un ou plusieurs radicaux nitro ou halogènes, ou
   - à déméthyler un composé intermédiaire (I'_{c}) de formule 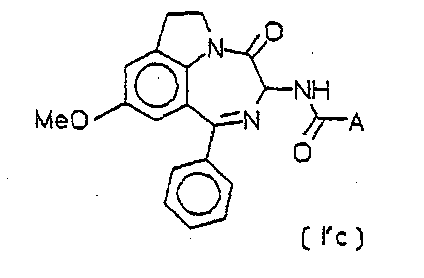 par un halogénure de bore ou d'aluminium, ou
   - à diazoter dans un premier temps un composé (Iₑ), de formule (I) dans lequel B est un groupe -NH₂, puis à hydrolyser dans un second temps le sel de diazonium intermédiaire, et qui consiste
**b**) pour obtenir les composés (Iₑ), de formule (I) dans laquelle B est un groupe -NH₂ :
   - à acyler un composé (IIₑ) de formule (II) dans laquelle B est un groupe -NH₂ par le réactif (III) défini précédemment en a), ou
   - à réduire le radical nitro d'un composé intermédiaire (I'_{d}) de formule 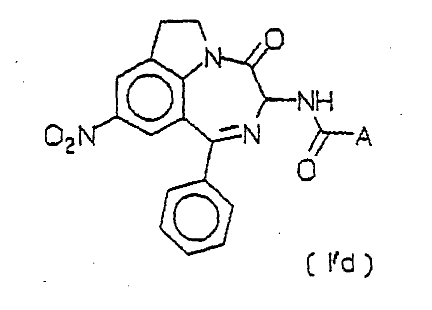 par l'action d'un métal tel que Zn ou Sn en milieu acide, ou celle d'un chlorure ou sulfure métallique tels que TiCl₃ ou Na₂S,
      et, comme montré au schéma 2 qui suit, qui consiste : 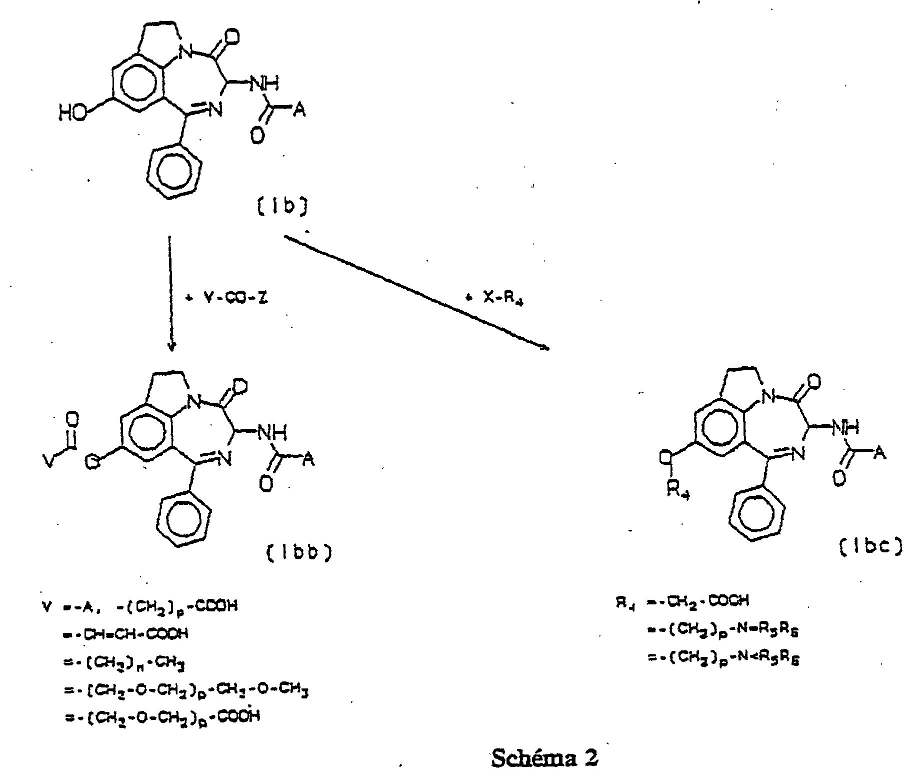
**c**) pour obtenir les composés (I_{bb}), de formule (I) dans laquelle B est un groupe -O-CO-V; V étant un groupe choisi parmi :
   i) - A, tel que défini précédemment en a),
   ii)- (CH₂)ₚ-CO₂H, où p est un entier égal à 2, 3 ou 4,
   iii) - CH = CH-CO₂H,
   iv) - (CH₂)ₙ-CH₃, où n est un entier égal ou supérieur à 0 et inférieur ou égal à 18,
   v) - (CH₂-O-CH₂)ₚ-CH₂-O-CH₃, où p est un entier égal à 2, 3 ou 4, ou
   vi) - (CH₂-O-CH₂)ₚ-CO₂H, où p est un entier égal à 2, 3 ou 4,
   à estérifier un composé (I_{b}), défini en a), par un réactif (III') de formule V-CO-Z dans laquelle Z a la signification précédemment définie en a), et qui consiste
**d**) pour obtenir les composés (I_{bc}), de formule (I) dans laquelle B est un groupe -O-R₄, R₄ étant choisi parmi :
   i) - CH₂-CO₂H,
   ii) - (CH₂)_{P}-NR₅R₆, où R₅ et R₆ sont indépendamment -H ou alkyle inférieur, ou
   iii) - (CH₂)ₚ-N-R₅-R₆, où R₅ et R₆ forment ensemble avec l'atome d'azote auquel ils sont reliés un hétérocycle,
   à faire réagir un composé (I_{b}), défini en a), avec une base forte telle qu'un hydrure de métal alcalin pour former un phénate, lequel est mis à réagir avec un halogénure XR₄ ; et, comme montré au schéma 3 qui suit, ce procédé de préparation consiste également :
**e)** pour obtenir les composés (Iₑₐ), de formule (I) dans laquelle B est un groupe -NH-C(NH)-NH₂, à faire réagir un composé (Iₑ), défini en b), avec un agent de guanylation tel que le cyanamide, et
**f)** pour obtenir les composés (I_{eb}), de formule (I) dans laquelle B est un groupe -NH-CO-V, V ayant la signification définie en c), à amidifier un composé (Iₑ), défini en b), par un réactif (III') : V-CO-Z défini en c) , et
**g)** pour obtenir les composés (I_{ec}), de formule (I) dans laquelle B est un groupe -NH-R₂, R₂ étant alkyle inférieur ou un groupe R₄ tel que défini en d), à faire réagir un composé (Iₑ), défini en b), en présence d'une base forte avec un halogénure d'alkyle XR₂, et
**h)** pour obtenir les composés (I_{ed}), de formule (I) dans laquelle B est un groupe -NR₂R₃, R₂ R₃ étant alkyles inférieurs, à pratiquer l'alkylation réductive d'un composé (I_{ec}), défini en g), par un aldéhyde R'₃CHO dans lequel R'₃ est l'homologue immédiatement inférieur de R₃, et enfin
**i)** pour obtenir les composés (Iₑₑ), de formule (I) dans laquelle B est un groupe -N-R₂-R₃, R₂ et R₃ formant un hétérocycle,
à effectuer une cyclisation par réaction d'un composé (Iₑ), défini en b), avec un réactif de formule

X-(CH₂)ₗ-Q-(CH₂)ₘ-X'

dans laquelle X et X', semblables ou différents, sont halogènes, Q est :
- une liaison simple de valence, et 1 et m sont des entiers allant de 1 à 3 avec I + m supérieur ou égal à 4 et inférieur ou égal à 6,
- un oxygène, un soufre ou un groupe -NH-, auquel cas 1 et m sont des entiers allant de 1 à 3 avec 1 + m supérieur ou égal à 3 et inférieur ou égal à 5, ou, de façon alternative, à alkyler une amino diazépino-indole intermédiaire (IIf) de formule (II) dans laquelle B est un groupe -N-R₂-R₃, R₂ et R₃ formant avec l'atome d'azote un hétérocycle, par un réactif (III) de formule Z-CO-A tel que précédemment défini.

Plus précisement la préparation de diazépino-indoles de formule (I) fait notamment appel à des réactions d'acylation, en particulier pour obtenir directement les composés (I_{b}) et (Iₑ) à partir des amines intermédiaires (II_{b}) et (IIₑ) (schéma 1) et d'obtenir les composés(Iₑₑ) à partir des intermédiaires (II_{f}) tel que montré au schéma 4bis . Pour réaliser cette étape, trois procédés, nommés A, B, et C, sont préférés. Ils se distinguent en particulier par la nature de l'agent acylant (III), de formule Z-CO-A, engagé. Ainsi Z représente selon les cas :
- un halogène X lorsque, dans le procédé "A", on engage un agent d'acylation (III_{A}) de formule X-CO-A ;
- un groupe pentafluorophényloxy lorsque, dans le procédé "B", on engage un agent d'acylation (III_{B}) de formule C₆F₅-O-CO-A ;
- un radical hydroxy lorsque, dans le procédé "C" , on engage un agent d'acylation (III_{C}) de formule A-COOH.

La réaction d'acylation, selon ces trois procédés, est conduite dans un solvant organique anhydre comme par exemple un hydrocarbure chloré comme du dichlorométhane ou du chloroforme, un éther-oxyde linéaire ou cyclique comme du diméthoxy-1, 2-éthane, du tétrahydrofurane ou du dioxane, un solvant polaire aprotique tel que la pyridine, du diméthylsulfoxyde ou du diméthylformamide ou tout autre solvant convenable, ou encore un mélange de plusieurs de ces solvants.

Avantageusement, on déplace favorablement la réaction par ajout d'une base et/ou d'un agent de condensation comme une carbodiimide N,N' disubstituée, le N,N'-carbonyldiimidazole, ou comme de préférence le tétrafluoroborate de O-[(éthoxycarbonyl)cyanométhylamino]-N,N,N',N'-tétraméthyluronium ou encore l'hexafluorophosphate de bromo-tris-pyrrolidino-phosphonium.

Les conditions opératoires des procédés A, B et C sont détaillées au début de la partie chimique expérimentale. 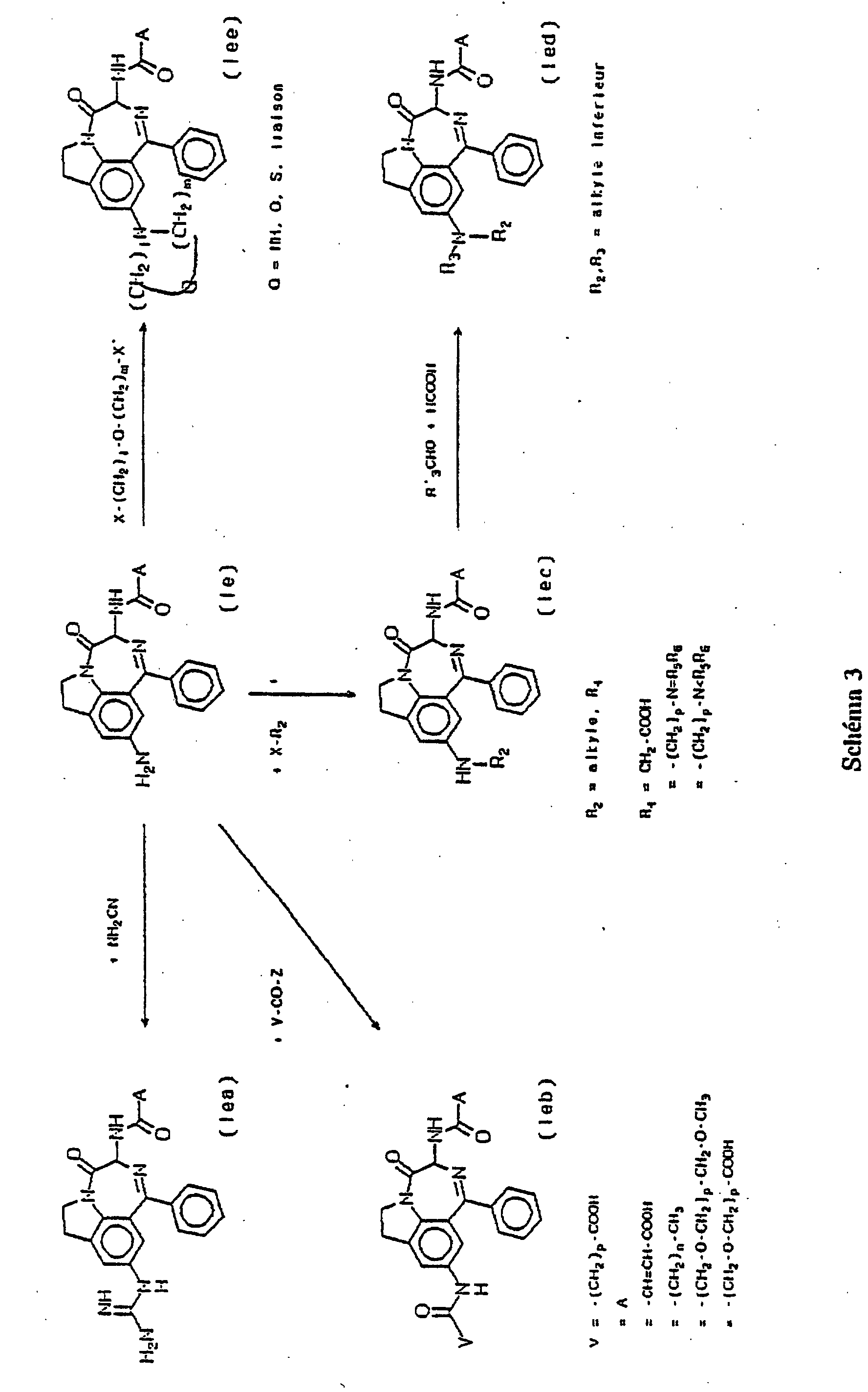 Le schéma 1 présente deux voies alternatives d'accès aux composés (I_{b}):
- l'une consiste à O-déméthyler par un halogénure de bore ou d'aluminium un composé intermédiaire (I'_{c}), dont la fonction phénol était protégée par un groupe méthyle ; l'acide de-Lewis préféré pour cette réaction est le tribromure de bore, auquel cas on opère à une température initiale comprise entre - 70 et - 20 °C ;
- l'autre consiste à diazoter un composé (Iₑ) avec l'acide nitreux en présence d'acide minéral puis, dans un second temps, à hydrolyser par chauffage le sel de diazonium obtenu, de préférence en présence d'un sel de cuivre.

Le schéma 1 indique que le composé (Iₑ) peut également être obtenu par réduction du groupe nitro d'un composé intermédiaire (I'_{d}), notamment par TiCl₃ ou Na₂S.

Le schéma 2 montre le procédé d'estérification conduisant aux diazépino-indoles de formule (I_{bb}), qui consiste à faire réagir un produit de formule (I_{b}) avec un dérivé d'acide carboxylique de formule V-(CO)-Z, dans laquelle Z a la signification indiquée ci-dessus et V a la signification indiquée au schéma 2.

Un procédé d'éthérification, représenté au schéma 2 et conduisant aux diazépino-indoles de formule (I_{bc}), consiste à faire réagir un produit de formule (I_{b}) avec une base forte telle que l'hydrure de sodium pour former un phénate, lequel est mis à réagir avec un halogénure d'alkyle XR₄, dans lequel R₄ est un radical alkyle substitué par une fonction acide carboxylique, notamment le radical -CH₂-CO₂H sous une forme protégée, ou bien R₄ est un radical alkyle substitué par une fonction amine terminale, notamment le radical-(CH₂)ₚ-NR₅R₆, R₅ et R₆ étant indépendamment -H ou alkyle inférieur, ou R₅ et R₆ formant, ensemble avec l'atome d'azote auquel ils sont reliés, un hétérocycle saturé de cinq à sept chaînons pouvant comprendre, comme hétéroatome non relié directement à l'atome d'azote, un oxygène, un soufre ou un azote ; et p étant un entier égal à 2, 3, ou 4. Lorsque R₄ est porteur d'une fonction acide carboxylique, celle-ci est protégée : on utilise par exemple l'α-bromo-acétate de tertiobutyle; après réaction avec le phénate, le produit obtenu est déprotégé par l'acide trifluoroacétique pour donner le produit (I_{bc}) où R₄= -CH₂CO2H.

Le schéma 3 présente :
- un procédé de préparation de diazépino-indoles de formule (Iₑₐ) qui consiste à faire réagir un diazépino-indole de formule (Iₑ) avec le cyanamide dans l'acétonitrile ; alternativement, on peut utiliser comme agent de guanylation le *N*-amidino-pyrazole, ou son dérivé 3,5-diméthyle;
- un procédé de préparation de diazépino-indoles de formule (I_{eb}) qui consiste à faire réagir un diazépino-indole (Iₑ) avec un dérivé d'acide carboxylique de formule V-(CO)-Z dans laquelle Z a la signification indiquée ci-dessus et V de signification indiqué au schéma 3, selon un des procédés A, B, C décrits ci-dessus et, de préférence, le procédé C;
- un procédé de préparation de diazépino-indoles de formule (I_{ec}) qui consiste à faire réagir un produit de formule (Iₑ) en présence d'une base forte telle que l'hydrure de sodium pour former un amidure, lequel est mis à réagir avec un halogénure d'alkyle XR₂,dans lequel R₂ a ia signification indiquée au schéma 3. Lorsque R₂ = R₄ = -(CH₂)ₚ-NR₅R₆, on utilise XR₄ sous forme de chlorhydrate;
- un procédé de préparation de diazépino-indoles de formule (I_{ed}) qui consiste à pratiquer l'alkylation réductive d'un diazépino-indole (I_{ec}), lequel est mis à réagir avec un aldéhyde R'₃CHO dans lequel R'₃ est l'homologue immédiatement inférieur de R₃, en présence d'acide formique ou acétique et d'un hydrure, de préférence NaBH₄ ou NaBH₃CN; quand on engage (Iₑ) dans cette réaction, on obtient directement un produit (I_{ed}) dans lequel B = -NR₂R₃, avec R₂ = R₃;
- un procédé de préparation de diazépino-indoles de formule (Iₑₑ) qui consiste à effectuer une cyclisation par réaction d'un composé (Iₑ) sur un réactif de formule X-(CH₂)ᵣ-Q-(CH₂)ₘ)-X' dans laquelle X et X', semblables ou différents, sont halogènes et de préférence le brome, Q est une liaison simple de valence et 1 et m sont des entiers de 1 à 3 et dont la somme va de 4 à 6; ou bien Q est -O-, -S-, ou -NH- auquel cas la somme 1 + m va de 3 à 5.

### Intermédiaires de formule (I'_{c}) et (I'_{d})

Les procédés A, B, ou de préférence C , déjà décrits, permettent, comme indiqué au schéma 1, à partir des intermédiaires (II_{c}) ou (II_{d}), de formule (II) où B représente respectivement un groupe -OCH₃ ou -NO₂, d'obtenir les intermédiaires (I'_{c}) ou (I'_{d}).

Le procédé général de préparation des amines intermédiaires (II) est illustré sur le schéma 4 qui suit et détaillé ci-après.

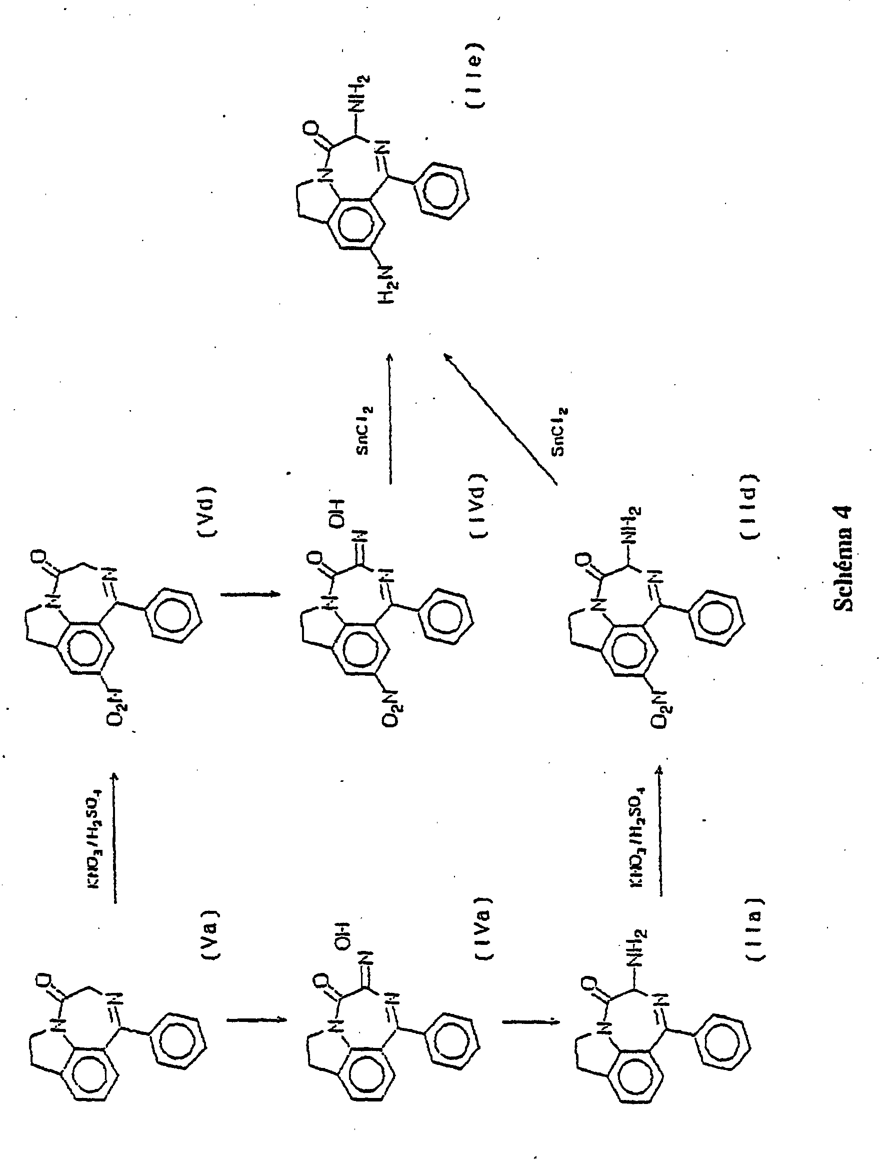

### Intermédiaires de formule (IIₑ) et (II_{d})

On peut préparer l'amine (IIₑ) en nitrant la position 9 d'un diazépino-indole (IIₐ) par le nitrate de potassium en milieu sulfurique. On obtient preférentiellement (II_{d}) dont la fonction -NO₂ peut être réduite en -NH₂ par de nombreux réducteurs, dont SnCl₂.

Une variante pour la préparation de l'intermédiaire (IIₑ) consiste à nitrer un diazépino-indole de formule (Vₐ). On fait ensuite réagir sur le composé (V_{d}) obtenu un réactif d'oximation pour obtenir l'oxime (IV_{d}). La réduction de (IV_{d}) par de l'hydrogène en présence d'un catalyseur de réduction ou par réaction avec du zinc en présence d'un acide permet de réduire les fonctions oxime et nitro pour donner (IIₑ).

### Intermédiaire de formule (II_{f})

Les intermédiaires sont préparés tel que montré au schéma 4bis qui suit en effectuant successivement :
i) - la protection de la fonction amine, d'un intermédiaire nitré (II_{d}) pour obtenir un intermédiaire (II_{dp}) comprenant par exemple un groupe t.butyloxycarbonyl (t.Boc), puis
ii) - à réduire le groupe nitro de cet intermédiaire par une hydrogènation qui par exemple peut être catalysée par le ruthénium pour obtenir un intermédiaire amine (IIₑₚ), puis
iii) - à alkyler la fonction amine obtenue par un réactif de formule X-(CH₂)ₗ-Q-(CH₂)ₘ-X' précédemment défini pour obtenir par cyclisation une amine hétérocyclique (II_{fp}), puis
iv) - à éliminer la protection introduite au stade i) de cette préparation en faisant réagir pour l'exemple d'une protection t.Boc de l'acide trifluoroacétique en milieu anhydre pour obtenir l'intermédiaire (II_{f}).

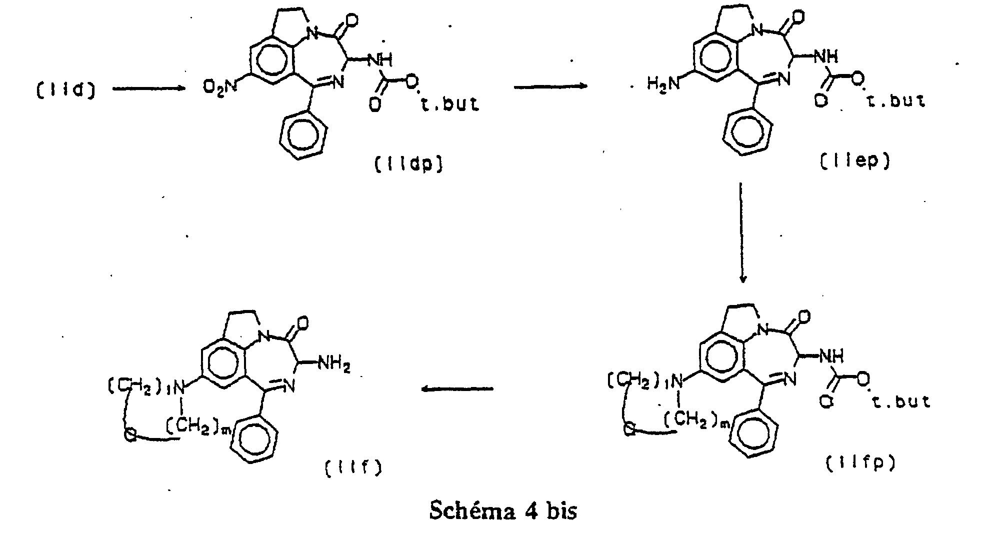

### Intermédiaire de formule (IIₐ)

Le procédé général de préparation des amines intermédiaires (IIₐ) sous leurs formes racémique et/ou d'énantiomère est documenté dans l'art antérieur. Par exemple, on peut préparer une amine de formule (IIₐ) en aminant en position alpha du carbonyle un diazépino-indole de formule (Vₐ) par un dérivé d'hydroxylamine ou par la chloramine ; ou encore, en deux étapes, en faisant réagir un composé de formule (Vₐ) sur un réactif d'oximation pour obtenir l'oxime de formule (IVₐ), la seconde étape consistant à réduire l'oxime catalytiquement par de l'hydrogène en présence d'un catalyseur de réduction ou par réaction avec du zinc en présence d'acide acétique ou avec du chlorure stanneux en présence d'acide chlorhydrique, pour obtenir le dérivé aminé (IIₐ).

### Intermédiaires de formule (II_{b}) et (II_{c}) - Schéma 5

A partir d'un diazépino-indole de formule (V_{c}), on prépare en deux étapes, avec un intermédiaire oxime (IV_{c}) et selon un procédé essentiellement identique à celui utilisé pour préparer (IIₐ), le composé (II_{c}). Par action du tribromure de bore, on déméthyle (II_{c}) en l'intermédiaire (II_{b}).

Le schéma 6 qui suit illustre le procédé de synthèse de (V_{c}) :

L'indole (IX) est réduit en l'indoline (VIII) correspondante, laquelle est condensée avec le benzonitrile (VII) en présence d'acide de Lewis pour donner, après hydrolyse, la benzophénone (VI).

La préparation, à partir de (VI) en présence de glycinate d'éthyle dans la pyridine, du produit de formule (V_{c}) est adaptée de la méthode décrite (méthode N) par Hester J.B. et al., 1970, J. Med. Chem. 13 : 827-835.

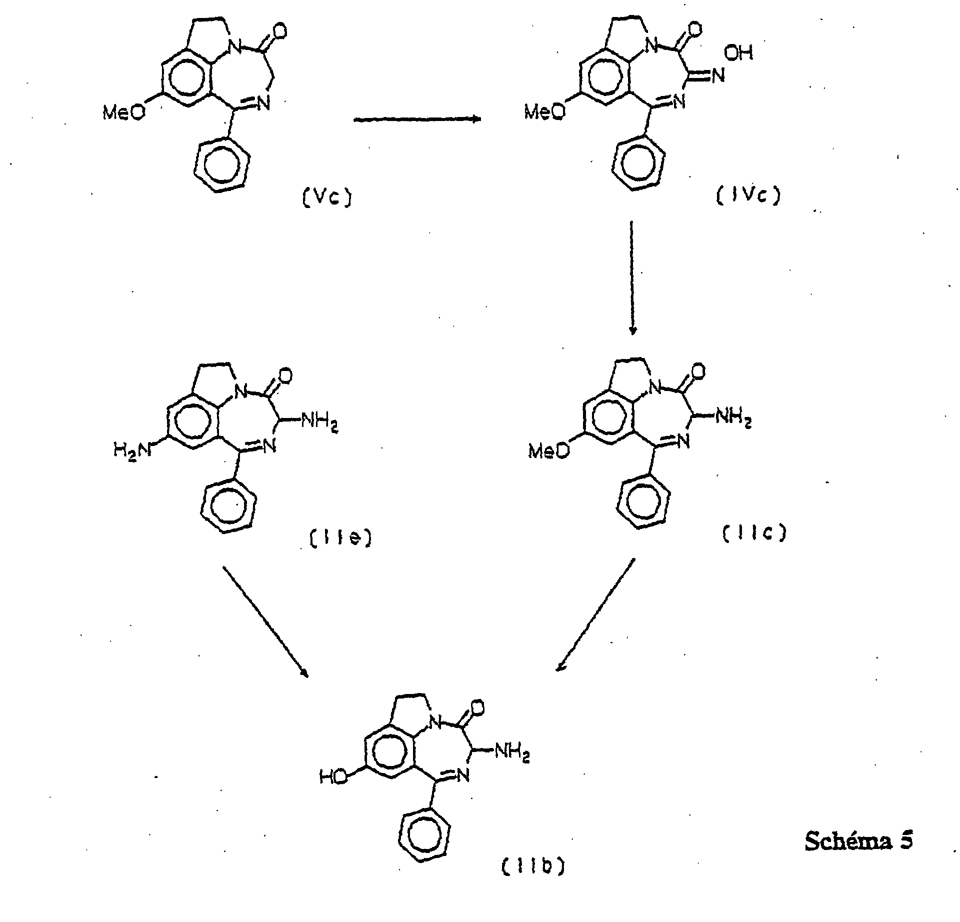

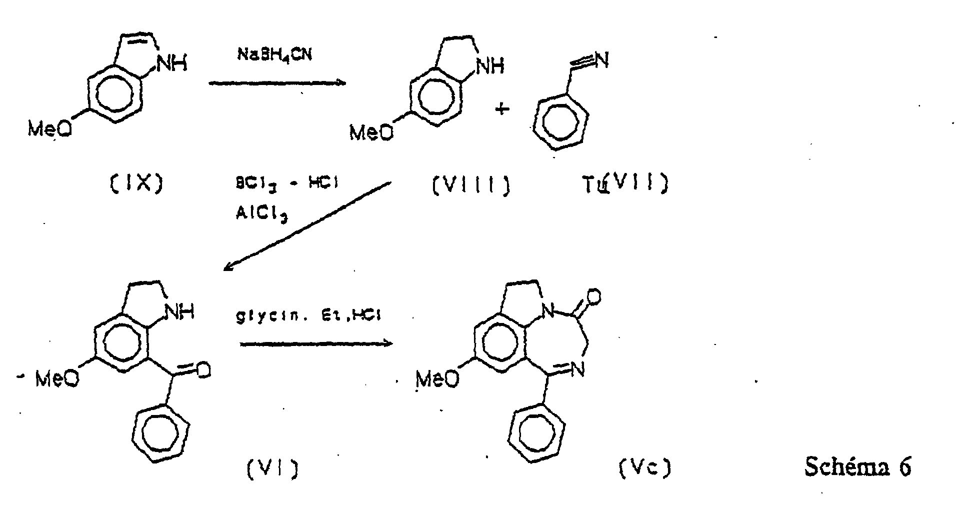

Entre autres possibilités, pour préparer un composé de formule (II) optiquement actif, on peut :
- condenser un composé (II) racémique avec un dérivé d'acide alpha aminé appartenant à la série D ou à la série L et dans lequel la fonction amine est protégée par un groupement facilement labile, de préférence le groupement tertiobutyloxycarbonyle.
   Le composé obtenu est déprotégé par hydrolyse, de préférence en milieu acide en présence d'acide trifluoro-acétique, et le produit obtenu est séparé en ses diastéréoisomères par chromatographie ; on obtient les deux isomères de l'amine condensée avec l'acide aminé.
   Par dégradation d'Edman, on revient ensuite aux deux énandomères de l'amine (II) ; ou encore,
- dissoudre un composé (II) racémique dans une solution d'acide optiquement actif, comme par exemple un énantiomère des acides mandélique, dibenzoyltartrique, di-*p*-toluyltartrique, camphosulfonique, *p*-nitrobenzoylglutamique, ou tartrique, pour former deux sels diastéréoisomères, puis par différence de solubilité, en cristalliser sélectivement un dans un solvant approprié.

Les produits intermédiaires de formule (IV) et les produits de formule (II) sont des intermédiaires utiles pour la préparation des produits actifs suivant l'invention.

L'invention vise également un médicament pour lutter contre les maladies inflammatoires, allergiques, contre la bronchoconstriction, ou utile dans le traitement de l'asthme, caractérisé en ce qu'il comprend un diazépino-indole suivant l'invention, sous une forme pharmaceutique adaptée à l'affection à traiter.

### Partie expérimentale

### Partie chimique

Les exemples suivants illustrent, sans pour autant la limiter, la mise en oeuvre des procédés et les produits de l'invention.La pureté, l'identité et les caractéristiques physico chimiques des produits et des intermédiaires essentiels préparés sont déterminées, ainsi :
- la pureté est vérifiée par chromatographies sur couches minces de gel de silice (Merck 60 - F254) et le Rf observé est rapporté pour le solvant d'élution utilisé qui est, le plus fréquemment, identique à celui utilisé pour la purification chromatographique préparative des composés. Ces solvants sont identifiés par les sigles suivants :
   S.A : dichlorométhane,
   S.A1 : dichiorométhane - acétone , 97 - 3 (v/v),
   S.A2 : dichlorométhane - acétone , 96 - 4 (v/v),
   S.A3 : dichlorométhane - acétone , 95 - 5 (v/v),
   S.A4 : dichlorométhane - acétone , 90 -10 (v/v),
   S.A5 : dichlorométhane - acétone , 88 -12 (v/v),
   S.A6: dichlorométhane - acétone , 85 -15 (v/v),
   S.A7 : dichlorométhane - acétate d'éthyle , 98 - 2 (v/v),
   S.A8 : dichlorométhane - méthanol , 98 - 2 (v/v),
   S.A9 : dichlorométhane - méthanol , 97 - 3 (v/v),
   S.A10 : dichiorométhane - méthanol , 95 - 5 (v/v),
   S.B : acétate d'éthyle,
   S.B1 : acétate d'éthyle - cyclohexane , 70 - 30 (v/v),
   S.B2 : acétate d'éthyle - cyclohexane, 60 - 40 (v/v),
   S.B3 : acétate d'éthyle - méthanol , 97 - 3 (v/v),
   S.B4 : acétate d'éthyle - méthanol, 95 - 5 (v/v),
   S.C : méthanol,
- l'identité de formule élémentaire des composés obtenus avec celle des structures visées est vérifiée par l'analyse des principaux éléments. Les résultats ne sont pas reportés mais indiqués conformes avec la structure proposée compte tenu d'éventuels solvats ou hydrates.
- l'identité des produits obtenus avec les structures proposées est vérifiée par leur spectre de résonance magnétique nucléaire du proton et par leur spectrographie infra-rouge.

Les spectres R.M.N ¹H sont réalisés à 400 MHz sur un appareil de marque Brüker, les composés étant dissous dans le deutérochloroforme avec le tétraméthylsilane comme référence interne. La nature des signaux, leurs déplacements chimiques en ppm, le nombre de protons qu'ils représentent et leur capacité d'échange avec D₂O sont notés.

Les spectres infra rouge sont enregistrés en pastille de bromure de potassium sur un spectromètre Shimadzu IR-435.
- les caractéristiques physico chimiques relevées sont leur point de fusion, déterminés par la méthode du tube capillaire et dont les valeurs rapportées ne sont pas corrigées, leur pouvoir rotatoire, déterminés à la température ambiante voisine de 20°C sur un appareil Polartronic en cuve de 10 cm de long et dont les résultats permettent dans certains cas d'apprécier la pureté optique par calcul de l'excès énantiomérique (e.e.).

Dans un but de normalisation, la nomenclature chimique des produits exemplifiés est celle déterminée à l'aide du logiciel "Autonom" version 1.0 (Beilstein Institut - Ed. Springler) qui génère les nomenclatures systématiques des composés selon les règles de l'IUPAC.

Tel que décrit précédemment, la préparation des composés (I) de l'invention met en oeuvre la réaction des amines intermédiaires (II) avec des halogénures (III_{A}) selon le procédé A, des esters, notamment pentafluorophényliques (III_{B}) selon le procédé B, ou des acides carboxyliques (III_{C}) selon le procédé C. Les modes opératoires généraux de ces procédés sont les suivants.

Procédé A : dans un réacteur protégé de l'humidité on dissout sous agitation 10,0 mmol d'une amine intermédiaire (II) dans 60 ml de dichlorométhane anhydre. A une température voisine de 20°C on ajoute alors 10,0 mmol d'halogénure d'acide (III_{A}) puis, goutte à goutte, 10,0 mmol de triéthylamine. La réaction est poursuivie sous agitation à la température ambiante comprise entre 15 et 25°C et son avancement est suivi par chromatographie sur couches minces. Lorsque la réaction est considérée terminée, on ajoute 120 ml de dichlorométhane au milieu réactionnel, le mélange est extrait successivement par 60 ml de solution HCl 1N, 60 ml de solution saturée de bicarbonate de sodium et finalement 60 ml d'eau. Après séchage le dichlorométhane est évaporé sous pression réduite, le résidu est purifié par chromatographie rapide sur une colonne de silice, selon une méthode adaptée de Still *et al*. (1978) J. Org. Chem. 43 : 2923, le solvant d'élution étant un mélange de polarité croissante, constitué, par exemple, d'acétone dans le dichlorométhane. Les fractions d'élution déterminées contenir le composé pur sont réunies puis évaporées sous pression réduite. Le produit purifié résiduel est soumis aux déterminations structurales et de pureté précédemment décrites.

- Procédé B :
stade 1 : dans 25 ml de dichlorométhane on dissout 10,0 mmol d'un acide intermédiaire (III_{c}) de formule A-COOH et 3,55 g (19,3 mmol) de pentafluorophénol. On ajoute ensuite 0,81 g (2,6 mmol) de para toluène sulfonate de para-diméthylamino-pyridinium et :
- soit 22,4 mmol de dicyclohexylcarbodiimide dans le procédé "B.a",
- soit 22,4 mmol de N-(3-diméthylaminopropyl)-N'-éthyl-carbodiimide dans le procédé "B.b".

Le mélange est agité durant 16 heures à la température du laboratoire voisine de 20°C puis l'insoluble est filtré. Le solvant est éliminé par distillation, le résidu est purifié par la technique de chromatographie rapide sur une colonne de silice en utilisant le plus couramment comme solvant d'élution un gradient d'acétone dans le dichlorométhane. Les fractions déterminées pures par C.C.M sont réunies, le solvant est évaporé et après analyse, l'ester intermédiaire (III_{B}) résiduel, sous forme de mousse amorphe, est utilisé tel quel dans le stade suivant.

stade 2 : 10,0 mmol de l'ester pentafluorophényle (III_{B}) préparé au stade précédent sont ajoutés à 10,0 mmol d'amine intermédiaire (II) dissous dans de l'acétate d'éthyle anhydre. Après 16 heures d'agitation à la température ambiante voisine de 20°C on filtre l'insoluble, évapore l'acétate d'éthyle sous vide puis purifie le résidu par la technique de chromatographie rapide sur une colonne de silice en utilisant le plus couramment comme solvant d'élution un gradient de méthanol dans le dichlorométhane. Les fractions déterminées pures par C.C.M sont réunies, le solvant est évaporé et le résidu purifié est identifié et analysé.

Procédé C (préféré) : dans un réacteur protégé de l'humidité on dissout sous agitation 10,0 mmol d'une amine intermédiaire (II) dans 50,0 ml de dichlorométhane anhydre. A la température du laboratoire voisine de 20°C on ajoute alors 11,0 mmol d'un acide intermédiaire (III_{c}) de formule A-COOH puis 10,0 mmol (3,28 g) de "TOTU" (dénomination abrégée pour tétrafluoroborate de O-[(Emoxycarbonyl)cyanométhylamino]-N,N,N',N'-tétraméthyluronium - fournisseur Fluka réf. 02580). Le mélange est refroidi à 0°C on ajoute alors 20,0 mmol (2,55 g) de N,N-diisopropyl-éthylamine après quoi le mélange est agité durant 12 heures à la température ambiante puis extrait successivement par 50 ml de solution HCl 1N, 50 ml d'une solution saturée de bicarbonate de sodium et finalement 50 ml d'eau.

Le solvant est évaporé sous vide et le résidu purifié par la technique de chromatographie rapide sur une colonne de silice en utilisant le plus couramment comme solvant d'élution un gradient de méthanol dans le dichiorométhane. Les fractions déterminées pures par C.C.M sont réunies, le solvant est évaporé et le résidu purifié est identifié et analysé.

### Composés intermédiaires (II)

### Intermédiaire1:(3R)-3-Amino-1-phényl-6,7-dihydro-3H-[1,4]diazépino[6,7,1-hi]indol-4-one [(IIa)-R; B = H]

La préparation du composé sous sa forme racémique est décrite à l'exemple 1 stades a) et b) de EP 0 340 064 Al. De même celle de l'énantiomère (R) est décrite à l'exemple 5 stades a) b) c) g) h) de la même demande. Toutefois, une méthode alternative qui consiste au dédoublement du composé racémique par la formation et la séparation de diastéréoisomères avec la N-acétyl-L-phényl-alanine est préférée, elle est rapportée ci-dessous:
- 74,0 g (267 mmol) de (3R,S)-3-Amino-1-phényl-6,7-dihydro-3H-[1,4]diazépino[6,7,1-hi]indol-4-one sont dissous dans 210 ml de n-propanol bouillant. Par ailleurs 44,1g (267 mmol) de N-acétyl-L-phényl-alanine sont dissous dans 140 ml de n-propanol bouillant. On mélange les deux solutions, laisse refroidir, amorce par quelques cristaux. Après trois jours de repos les cristaux sont filtrés et séchés. Poids : 50,0 g (e.e. = 77%).

Le produit est recristallisé à deux reprises successives dans l'acétate d'éthyle bouillant. On obtient 39,0 g (e.e. = 97%). Les eaux mères de la première cristallisation sont évaporées, le résidu repris par de l'acétate d'êthyle bouillant. Après cristallisation, filtration et séchage on obtient 35,0 g de cristaux (e.e. = 50%), qui, après deux cristallisations successives dans l'acétate d'éthyle bouillant permettent d'obtenir 17,0 g (e.e. = 97%) de produit. Les deux jets réunis représentent 56,0 g (Rdt = 95 %) de sel de l'énantiomère 3R de l'amine avec la N-acétyl-L-phényl-alanine. F = 171°C. [α]_{D} = +132°(c = 1, méthanol).
- 42,4 g (96 mmol) du sel de l'amine (3R) sont agités violemment en présence de 500ml d'acétate d'éthyle et de 500ml de soude normale. Après dissolution, la phase acétate d'éthyle est séparée, lavée par de l'eau saturée de chlorure de sodium, puis déshydratée et évaporée. On obtient 25,4 g de l'amine attendue. Rdt = 95%. F =79°C. [α]_{D} = 172° (c = 1, CH₂Cl₂).

R.M.N.¹H δ(ppm): 3,05-3,5(m,2H); 3,3(s.large, 2H éch.); 3,9-4,0(m, 1H); 4,6-4,7 (m, 1H); 7,05-7,6 (m, 9H).

I.R.:3350, 1670, 1600, 1560, 1420, 1380, 1340, 1290, 1240, 760, 730, 690^{cm-1}.

### Intermédiaire 2 : (3R)-3,9-diamino-1-phényl-6,7-dihydro-3H-[1,4]diazépino[6,7,1-hi]indol-4-one [(IIe) - R ; B = -NR₂R₃; R₂ = R₃ = H].

- Stade-1 : Dans un réacteur de 100 ml, on introduit 51,0 ml d'acide sulfurique concentré (d = 1,83) et sous agitation on ajoute 16,0 g (57,7 mmol) de (3R)-3-Amino-1-phényl-6,7-dihydro-3H-[1,4]diazépino[6,7,1-hi]indol-4-one, intermédiaire 1 précédemment décrit.

Durant l'introduction exothermique la température atteint 70 °C, lasolution marron obtenue est refroidie à 5-10 °C. On introduit alors rapidement 6,93 g (68,5 mmol) de nitrate de potassium pur dissous dans 17,0 ml d'acide sulfurique (d = 1,83). La température progresse à 40 °C puis on maintient 40 minutes sous agitation à 20 °C.

La solution brune est précipitée dans 600 ml d'un mélange eau et glace. Le mélange est alcalinisé avec une solution d'ammoniaque concentré puis extrait par 3 fois 150 ml de dichlorométhane. les phases organiques sont lavées à l'eau, déshydratées, les solvants sont ensuite éliminés par distillation. On obtient un résidu meringué marron clair (17,5 g) qui est purifié par chromatographie rapide sur silice. L'élution par du dichlorométhane progressivement enrichi en méthanol permet d'obtenir 12,0 g de (3R)-3-amino-9-nitro-1-phényl-6,7-dihydro-3H-[1,4]diazépino[6,7,1-hi]indol-4-one [(IId) - R; B = NO₂] purifié.

Rdt = 75 % F = 177-178 °C [α]_{D} = + 66,8 °C (c = 0,4, CH₂Cl₂)
- Stade-2 : Dans un réacteur de 250 ml, on introduit 13,0 g (40,3 mmol) du dérivé nitré obtenu au stade précédent, 45,5 g (202 mmol) de chlorure d'étain dihydrate et 81 ml d'éthanol. Sous agitation le mélange est porté à 70 °C et maintenu 30 minutes à cette température. On obtient une solution marron et on distille alors environ 60 ml de solvant. On reprend le résidu par 400 ml d'eau glacée et extrait à l'éther la phase aqueuse et la gomme insoluble.

La phase éthérée est écartée. La phase aqueuse et la gomme sont alcanisées par une solution de soude jusqu'à pH 12. Le mélange est extrait par 3 fois 150 ml de dichlorométhane. Les phases organiques réunies sont lavées, séchées puis le dichlorométhane est éliminé par distillation. Le résidu (13, 0 g) est purifié par chromatographie sur colonne de silice en éluant par du dichlorométhane progressivement enrichi en méthanol. On obtient 12,4 g de (3R)-3,9-diamino-1-phényl-6,7-dihydro-3H-[1,4]diazépino[6,7,1-hi]indol-4-one [intermédiaire (IIₑ)-R ; B = -NR₂R₃; R₂ = R₃ = H].

R.M.N.¹H δ(ppm): 7,5-7,65 (m,2H); 7,5-7,3 (m,3H); 6,8 (d,1H); 6,4 (d,1H); 4,5-4,65 (m,1H); 3,8-4 (q,1H); 3,15-3,3 (m,1H); 2,95-3,05 (m,1H); 2,50-4,00 (m, 2H)
I.R. : 3300, 3200; 1660, 1580, 1480, 1440, 1360, 1240, 710 cm⁻¹

### Intermédiaires 3

### -3.a:(3R,S)-3-Amino-9-méthoxy-1-phényl-6,7-dihydro-3H-[1,4]diazépinol[6,7,1-hi]indol-4-one. [(II_{c}) - R,S; B = CH₃O]

Le composé est préparé à partir de 5-méthoxyindole, en 5 stades.
- Stade-1 : 5-Méthoxyindoline.

A une solution de 50,0 g (340 mmol) de 5-méthoxyindole dans 700 ml d'acide acétique glacial, on ajoute à 15-20°C par petites quantités 53,4 g (850 mmol) de cyanoborohydrure de sodium. L'addition faiblement exothermique est effectuée en 3 heures et s'accompagne d'un léger dégagement d'hydrogène. On laisse 12 heures sous agitation en dessous de 20°C, puis ajoute 700 ml d'eau et ajuste le pH du milieu réactionnel entre 10 et 12 par addition de 1200 ml d'une solution de soude à 30%. Le mélange est extrait deux fois par du dichlorométhane, puis la phase organique est lavée avec 300 ml d'eau. On évapore, le résidu est purifié par chromatographie rapide sur une colonne de silice, l'éluant utilisé étant un mélange de polarité croissante de méthanol dans du dichlorométhane. On obtient 41,84 g d'huile incolore qui devient jaune clair au stockage (à conserver sous atmosphère d'azote et à l'abri de la lumière).

Rdt = 83% - C.C.M.: S.B2; 0,38.

R.M.N.¹H δ(ppm): 3,00 (t, 2H); 3,40 (s, 1H éch.); 3,50(t, 2H); 3,70 (s, 3H); 6,60 (s, 2H);
6,80 (s, 1H).
- Stade-2 : 7-benzoyl-5-méthoxyindoline.

On dissout 5,00 g (33,5 mmol) de 5-méthoxylindoline dans 50 ml de 1,2-dichloroéthane. On ajoute goutte à goutte à t< 5°C 33,5 ml (33,5 mmol) de trichlorure de bore en solution molaire dans du dichlorométhane, 6,90 g (67 mmol) de benzonitrile. Le mélange est chauffé 6 heures au reflux (t, masse = 82-84°C). Après refroidissement l'hydrolyse est réalisée par ajout de 33,5 ml d'acide chlorhydrique 4N et chauffage durant 20 minutes à 80°C. On laisse refroidir vers 20°C et extrait par du dichlorométhane. La phase aqueuse est réextraite par 100 ml de dichlorométhane. On lave les phases organiques réunies par une solution de soude, puis par une solution concentrée de chlorure de sodium, et sèche sur sulfate de sodium. Après filtration et évaporation on obtient 3,12 g de solide jaune orangé.

Rdt= 38% - F =123°C - C.C.M.: S.A7; 0,81

R.M.N.¹H δ(ppm): 3,05 (t, 2H); 3,65 (s, 3H); 3,75 (t, 2H); 6,75 (s.large, 2H dont 1H éch.); 6,95 (s.large,1H); 7,40-7,55 (m, 3H); 7,65 (m, 2H)
- Stade-3 : 9-méthoxy-1-phényl-6,7-dihydro-3H-[1,4]diazépino[6,7,1-hi]indol-4-one.

Dans 75 ml de pyridine, on introduit 4,71 g (18,6 mmol) de 7-benzoyl-5-méthoxylindoline puis 16,2 g (116 mmol) de chlorhydrate de glycinate d'éthyle. On chauffe sous agitation à 110-115°C tout en distillant les fractions légères qui se forment. Après 12 heures on refroidit et ajoute 100 ml d'une solution de carbonate de sodium à 2,5% dans de l'eau et 100 ml de dichlorométhane. La phase aqueuse est séparée, extraite par 100 ml de dichlorométhane. Les phases organiques sont réunies et lavées à l'eau. Le solvant est évaporé puis le résidu purifié par chromatographie rapide sur une colonne de silice, l'éluant utilisé étant un gradient d'acétone dans le dichlorométhane. On obtient 4,15 g de produit purifié sous forme d'une résine brune.

Rdt = 82% - C.C.M.: S.A6; 0,73.

R.M.N.¹H δ(ppm): 3,10 (t, 2H); 3,70 (s, 3H); 4,30 (t, 2H); 3,90 (s, 2H); 6,60 (s, 1H); 7,00 (s, 1H); 7,30-7,50 (m, 3H); 7,60 (d, 2H).
- Stade-4 :3-hydroxyimino-9-méthoxy-1-phényl-6,7-dihydro-3H-[1,4]diazépino[6,7,1-hi] indol-4-one.

6,78 g (26 mmol) de produit précédent sont dissous dans un mélange de 26 ml de tétrahydrofurane et de 51 ml de toluène. On refroidit et ajoute à une température inférieure à 0°C, 7,29 g (65 mmol) de tertio-butylate de potassium. L'addition est exothermique et la solution se colore en noir. Après 20 minutes d'agitation on ajoute en 10 minutes environ 3,20 g (27,3 mmol) de nitrite d'isoamyle. On maintient 10 minutes sous agitation en dessous de 0°C, puis ajoute 10,3 ml d'acide acétique glacial et 100 ml d'eau. On filtre un insoluble et ajoute 50 ml de dichlorométhane. On décante et lave la phase aqueuse avec 100 ml de dichlorométhane. Les phases organiques sont réunies et lavées par 100 ml d'eau. Après évaporation du solvant le résidu est purifié par chromatographie. On obtient 4,44 g d'un solide jaune orangé.

Rdt = 53% - F=205°C. ; C.C.M.: S.A5; 0,17.

R.M.N.¹H δ(ppm): 3,20 (t, 2H); 3,70 (s, 3H); 4,40 (t, 2H); 6,70 (t, 2H); 7,10 (s, 1H); 7,40-7,60 (m, 3H); 7,80 (d, 2H); 8,60 (s, 1H).
- Stade-5 : (3R,S)-3-Amino-9-méthoxy-1-phényl-6,7-dihydro-3H-[1,4]diazépino[6,7,1-hi] indol-4-one.

On ajoute 4,38 g de ruthénium à 5% sur charbon à une solution de 14,6 g (45 mmol) de produit obtenu au stade précédent dans 1,01 de méthanol. On hydrogène sous une pression de 8 bars à 80°C pendant 6 heures, puis filtre et rince le catalyseur. Après évaporation le résidu est purifié par chromatographie rapide sur une colonne de silice, l'éluant utilisé étant un mélange de dichlorométhane enrichi progressivement en méthanol. On obtient 9,66 g d'amine purifiée sous la forme d'un solide jaune-beige.

Rdt = 67% - F=84°C.; C.C.M.: S.B3; 0,24.

R.M.N.¹H δ(ppm): 3,20 (t, 2H); 3,70 (s, 3H); 4,40 (t, 2H); 5,30 (s, 1H); 6,70 (s, 1H); 7,10 (s, 1H); 7,40-7,80 (m, 5H); 8,10 et 8,50 (s, larges, 2H éch).
- **3.b**:(3R)-3-Amino-9-méthoxy-1-phényl-6,7-dihydro-3H-[1,4]diazépino[6,7,1-hi]indol-4-one [(II_{c}) - R; B = CH₃O]

10,0 g (32,3 mmol) de l'amine (R,S) intermédiaire 3.a sont dissous dans 100 ml d'acétonitrile au reflux. Par ailleurs, 12,47 g (32,3 mmol) d'acide di-para-toluoyl-tartrique sont dissous au reflux dans 100 ml d'acétonitrile. Les solutions chaudes sont mélangées puis abandonnées pour cristallisation par refroidissement à la température du laboratoire. Après une nuit de repos, on filtre les cristaux blancs et lave ceux-ci par 100 ml d'acétonitrile froid puis sèche. Ces cristaux (e.e. = 37 %) sont recristallisés à deux reprises successives dans de l'acétonitrile pour obtenir le produit purifié (e.e. =99,5 %). Cette purification est suivie par chromatographie sur une colonne optiquement active C₁₈ de type Pirckle, en éluant par un mélange 50/50 d'isopropanol et de n-hexane. On obtient 9,9 g de produit . Rdt = 44%. F=168°C.

Les 9,9 g du sel précédent sont mis en suspension dans 100 ml d'acétate d'éthyle. On ajoute sous forte agitation une solution saturée de bicarbonate de sodium, après quelques ' minutes on écarte la phase aqueuse. La phase organique est lavée par 50 ml d'eau, séchée, puis le solvant est évaporé à froid sous atmosphère d'azote. On obtient 4,1 g de base purifiée.

Rdt = 95% - F = 84 °C - [α]_{D} = + 23 ° (c = 1, CH₂Cl₂)
- **3.c** : 1 (3R) Isoquinoline-3-carboxylique acide (9-méthoxy-4-oxo-1-phényl-3,4,6,7-tétrahydro-[1,4]diazépino[6,7,1-hi]indol-3-yl)-amide

[(I'_{c}); A = 3-isoquinolyl, B = CH₃O]

Le composé est préparé tel que décrit au procédé général B avec l'intermédiaire 3.b précédent et l'intermédiaire ester pentafluorophénylique de l'acide isoquinoline-3-carboxylique.

Rdt = 87% - solide blanc -F= 211°C - [α]_{D}= +0,30° (c = 1, CH₂Cl₂); C.C.M.: S.B1; 0,30
R.M.N.¹H δ(ppm): 3,10 (m, 1H); 3,35 (m, 1H); 3,75 (s, 3H); 4,00 (m, 1H); 4,70 (m, 1H); 5,70 (d, 1H s. par éch.); 6,70 (s.large, 1H); 7,10 (s.large, 1H); 7,20-7,80 (m, 7H); 8,00 (d, 1H); 8,10 (d, 1H); 8,65 (s, 1H); 9,30 (s, 1H); 9,90 (d, 1H éch.).

I.R.: 3360, 1665, 1500, 1490, 1470,1345, 1265, 1225, 1145, 700 cm⁻¹.

### Intermédiaire 4 : (3R)-3-amino-1-phényl-9-(pyrrolidin-1-yl)6,7-dihydro-3H-[1,4]diazépino [6,7,1-hi]indol-4-one [(IIf) - R ; B = -NR₂R₃; -R2 - R₃ = -(CH₂)₄-].

- Stade-1 : i) - dans un réacteur protégé de l'humidité et sous atmosphère d'azote on introduit 13,0 g (40,3 mmol) de (3R)-3-amino-9-nitro-1-phényl-6,7-dihydro-3H-[1,4]diazépino[6,7,1-hi]indol-4-one [ composé (IId); B = NO₂ préparé à l'intermédiaire 2-stade 1] dans 78 ml de THF déshydraté sur tamis moléculaire. La solution est refroidie et à t < 5°C on ajoute sous agitation 10,56 g (48,4 mmol) de carbonate de t.butyle dans 40 ml de THF. On laisse la température remonter à 20-25°C et abandonne 16 heures au repos. Les solvants sont éliminés par distillation sur bain marie et sous vide, le résidu est concrétisé dans 100 ml d'éther de pétrole puis filtré et séché (Rdt 100%). Le(3R)-N-(9-nitro-4-oxo-1-phényl-6,7-dihydro-3H-[1,4]diazépino[6,7,1-hi]indol-3-yl)-t.butyloxycarbamide (IIdp) est engagé tel quel dans le stade suivant.
   ii) - dans un autoclave pour hydrogénation on introduit dans un litre de méthanol, 11,0 g (26 mmol) de l'intermédiaire obtenu au stade précédent et 4,0 g de charbon à 5% de ruthénium. L'appareil est mis sous atmosphère d'hydrogène et la réduction est réalisée sous agitation à 85°C durant 6 heures. Le catalyseur est filtré, le solvant est éliminé par distillation sous vide et le résidu purifié par chromatographie rapide sur colonne de silice.
   L'élution par le dichlorométhane contenant 2% (v/v) de méthanol permet d'obtenir après élimination des solvants, le (3R)-N-(9-amino-4-oxo-1-phényl-6,7-dihydro-3H-[1,4]diazépino[6,7,1-hi]indol-3-yl)-t.butyloxycarbamide (IIep) purifié, sous forme amorphe.
   Poids : 8,8 g - Rdt = 86% - CCM : S.A8 Rf : 0,40
- Stade-2 : dans un réacteur protégé de l'humidité on introduit dans 150 ml de diméthylformamide (DMF) anhydre, 6,0 g (15,3 mmol) de l'intermédiaire (IIep) précédent, 3,60 ml soit 6,60 g de 1,4 - dibromobutane (30,6 mmol) et 6,38 g (76 mmole) d'hydrogénocarbonate de sodium. La suspension est chauffée à 60°C sous agitation durant 7 heures puis le DMF est éliminé par distillation sous vide, le résidu est solubilisé dans 150 ml de dichlorométhane. La solution est extraite à l'eau puis séchée, le solvant distillé sous vide et le résidu purifié par chromatographie rapide sur colonne de silice. L'élution par le -dichlorométhane contenant 1% de méthanol (v/v) permet d'obtenir 4,10 g de (3R)N-(4-oxo-1-phényl-9-(pyrrolidin-1-yl)-6,7-dihydro-3H-[1,4]diazépino[6,7,1-hi]indol-3-yl)-t.butyloxy carbamide (IIfp) purifié. Rdt = 60% - CCM : S.A8 Rf : 0,60
- Stade-3 : dans un réacteur protégé de l'humidité on introduit dans 48 ml de dichlorométhane anhydre, 4,10 g (9,2 mmol) d'intermédiaire (IIfp) obtenu au stade précédent. A la solution obtenue, on ajoute goutte à goutte à température ambiante 24,75 ml, soit 36,34 g (321 mmol) d'acide trifluoroacétique pur (d=1,48). Le mélange est agité 2 heures à 20-25°C puis les solvants sont éliminés par distillation sous vide. Le résidu est purifié par chromatographie rapide sur colonne de silice, l'élution par le dichlorométhane contenant 5% (v/v) de méthanol permet d'obtenir après évaporation des solvants le produit purifié sous forme amorphe. Poids : 4,35 g. Le composé est salifié par l'acide trifluoroacétique. Il est traité par une solution saturée de bicarbonate de sodium et le mélange est extrait par le dichlorométhane. Après évaporation le (3R)-3-amino-1-phényl-9-(pyrrolidin-1-yl) 6,7-dihydro-3H-[1,4]diazépino [6,7,1-hi]indol-4-one[(IIf) est obtenu sous forme amorphe. Poids : 3,00 g ; Rdt = 71% - CCM : S.A8 Rf : 0,80

### Exemples de l'invention (I)

### Exemple 1 : (3R)Isoquinoline-3-carboxylique acide (9-hydroxy-4-oxo-1-phényl-3,4,6,7-tétrahydro-[1,4]diazépino[6,7,1-hi]indol-3-yl)-amide

[(I_{B}) ; A = 3-Isoquinolyl; B = -OR₁ , R₁ = H]

Dans un réacteur de 50 ml protégé de l'humidité et sous atmosphère d'azote, on dissout 1,00 g (2,2 mmol) de l'intermédiaire 3.C (3R)Isoquinoline-3-acide carboxylique (9-méthoxy-4-oxo-1-phényl-3,4,6,7-tetrahydro-[1,4]diazépino[6,7,1-hi]indol-3-yl)-amide dans 20,0 ml de dichlorométhane déshydraté sur tamis moléculaire.

La solution est refroidie à - 50 °C et, sous agitation, on ajoute rapidement 5,41 g (22 mmol) de tribromure de bore.

Le mélange, marron et hétérogène est agité à 20-25 °C durant 3 heures puis précipité dans un mélange de 50 ml d'eau et 30 ml de dichlorométhane. L'insoluble est filtré, la phase aqueuse écartée et le dichlorométhane évaporé.

Le résidu d'évaporation et l'insoluble sont réunis et purifiés par chromatographie sur colonne de silice. L'élution par le mélange S.A8 permet d'obtenir 0,48 g de produit purifié amorphe jaune - Rdt = 49, 5 %. - F = 215 °C

Analyse conforme pour C₂₇H₂₀N₄O₃, (0.15 CH₂Cl₂), (0.25H₂O) - C.C.M. : S.A1 ; 0,33 R.M.N.¹Hδ(ppm) : 3,10 (m,1H); 3,35 (m,1H); 3,95 (m,1H); 4,50 (m,1H); 5,40 (d,1H); 6,05 (d,1H); 7,1 (d,1H); 7,45 (m,5H); 7,90 (m,2H); 8,30 (m,2H); 8,65 (s,1H); 9,50 (s,1H); 9,70 (d,1H, éch. D₂O); 9,75 (s, 1H, éch. D₂O).

I.R.: 3450, 3200, 1680, 1660, 1620, 1590, 1510, 1460, 1440, 1370, 1320, 1270, 1230, 1140, 1100, 1060, 960, 860, 760, 740, 700, 630, 540, 490 cm⁻¹

### Exemple 2 : (3R)4-t-butyloxycarbonylamino-N-(9-amino-4-oxo-1-phényl-3,4,6,7-tétrahydro-[1,4]diazépino[6,7,1-hi]indol-3-yl)-benzamide

[(Iₑ) ; A = 4-t.butyloxycarbonylaminophényl ; B = -NR₂R₃ ; R₂ = R₃ = H]

Dans un réacteur protégé de l'humidité on dissout sous agitation 1,60 g (5,5 mmol) de (3R)-3,9-diamino-1-phény]-6,7-dihydro-3H-[1,4]diazépino[6,7,1-hi]indol-4-one (intermédiaire 2) dans 60,0 ml de dichlorométhane anhydre. A 20 °C environ on ajoute alors 1,44 g (6,05 mmol) d'acide 4-t.butyloxycarbonylaminobenzoïque puis 1,80 g (5,5 mmol) de "TOTU" (dénomination abrégée pour tétrafluoroborate de O-[(Ethoxycarbonyl)cyanométhylamino]-N,N,N',N'-tétraméthyluronium - fournisseur Fluka réf. 02580). Le mélange est refroidi à 0 ° C on ajoute alors 1,9 ml soit 1,42 g (11,0 mmol) de N, N diisopropyl-éthylamine après quoi le mélange est agité durant 12 heures à la température ambiante puis extrait successivement par 50 ml de solutions HCl 1N, 50 ml d'une solution saturée de bicarbonate de sodium et finalement 50 ml d'eau. Le solvant est évaporé sous vide et le résidu d'un poids de 2,75 g est purifié par la technique de chromatographie rapide sur une colonne de silice en utilisant comme solvant d'élution un mélange acétate d'éthyle - hexane 80-20 v/v. Les fractions déterminées pures par C.C.M. sont réunies, le solvant est évaporé et le résidu (1,78g) purifié est solubilisé dans 6 ml d'isopropanol puis précipité par addition de 100 ml d'hexane. Le produit est filtré, séché sous vide. Poids : 1,30g
Rdt = 46 % - Poudre jaune - F = 215-225 °C - [α]_{D} = + 47 ° (C = 1, CH₂Cl₂) Analyse conforme pour C₂₉H₂₉N₅O₄ - C.C.M. : S.B ; 0,45
R.M.N. ¹H δ (ppm) : 1,50 (s, 9H); 2,95-3,10 (m, 1H); 3,20-3,35 (m, 1H); 3,80 (s, 2H); 3,85-4,00 (m, 1H); 4,55-4,70 (m, 1H); 5,55-5,65 (d, 1H); 6,40 (d, 1H); 6,85 (d, 1H); 7,15 (s,1H); 7,30-7,60 (m, 7H); 7,80-7,95 (d, 2H); 7,95-8,05 (d, 1H).

I.R. : 3300, 2995, 1640, 1470, 1370, 1310, 1230, 1150, 1050, 700 cm⁻¹.

### Exemple 3 : (3R)4-amino-N-(9-amino-4-oxo-1-phényl-3,4,6,7-tétrahydro-[1,4]diazépino[6,7,1-hi]indol-3-yl)-benzamide

[(Iₑ) ; A = 4-aminophényl; B = -NR₂R₃ ; R₂ = R₃ = H]

Dans un réacteur de 50 ml on dissout sous agitation dans 30 ml de dichlorométhane anhydre, 1,00 g (1,95 mmole) de (3R)-4-t.butyloxycarbonylamino-N-(9-amino-4-oxo-1-phényl-3,4,6,7-tétrahydro[1,4]diazépino[6,7,1,-hi]indol-3-yl)-benzamide (produit de l'exemple 2).

La solution jaune pâle est refroidie à 0 °C et on ajoute sans dépasser 5 °C 10,0 ml d'acide trifluoroacétique (d = 1,480). Le milieu réactionnel qui devient rouge-orangé est maintenu 45 minutes à 0-5 °C sous agitation puis évaporé sous vide sur bain marie.

Le résidu est repris par 100 ml de dichlorométhane et lavé par une solution NaOH 2N puis à l'eau. Le solvant est évaporé ensuite sous vide et on obtient 0,85 g d'un résidu jaune que l'on purifie par chromatographie rapide sur colonne de silice en éluant par le mélange S.A8. Les fractions purifiées évaporées conduisent à 0,60 g de produit jaune que l'on cristallise à 20°C environ dans 20 ml d'isopropanol. L'insoluble est filtré, séché. Poids: 0,50 g.
Rdt = 60 % - Poudre jaune - F = 276 °C - [α]_{D} = + 63 ° (c = 1, MeOH)
Analyse conforme pour C₂₄H₂₁N₅O₂; (0.25 i.PrOH) - C.C.M. : S.A3; 0,20 R.M.N.¹Hδ(ppm): 2,95-3,10 (m,1H); 3,15-3,35 (m,1H); 3,75-3,90 (m,1H); 4,35-4,50 (m,1H); 5,30 (s,2H); 5,40-5,50 (d,1H); 5,75 (s,2H); 6,35 (d,1H); 6,55-6,65 (d,2H); 6,85 (d,1H); 7,40-7,60(m,5H); 7,70-7,80 (d,2H); 8,85-8,95 (d,1H).

I.R. : 3300, 1600, 1475, 1370, 1270, 1180, 830, 765, 695, 530 cm⁻¹

### Exemple 4 : (3R)4-amine-N-(9-amino-4-oxo-1-phényl-3,4,6,7-tétrahydro-[1,4]diazépino[6,7,1-hi]indol-3-yl)-3,5-dichloro--benzamide

[(Iₑ) ; A = 4-amino-3,5-dichloro-phényl; B = -NR₂R₃ ; R₂ = R₃ = H]

Le composé est préparé selon le mode opératoire de l'exemple 2, à partir de l'amine intermédiaire 2 et de l'acide 4-amino-3,5-dichlorobenzoïque.

Après réaction le résidu est purifié par chromatographie rapide sur colonne de silice en éluant avec le mélange S.A8. Le produit obtenu est finalement recristallisé dans l'acétate d'éthyle.

Rdt = 55 % - Poudre jaune - F = > 280 °C - [α]_{D} = + 48,5 ° (c = 1, MeOH)
Analyse conforme pour C₂₄H₁₉Cl₂N₅O₂
R.M.N.¹Hδ(ppm) : 2,85-3,05 (m,1H); 3,20-3,35 (m,1H); 3,80-3,90 (m,1H); 4,40-4,50 (m,1H); 5,20-5,30 (m,2H); 5,45 (d,1H); 6,10 (s,2H); 6,40 (s,1H); 6,90 (s,1H); 7,40-7,60 (m,5H); 8,00 (s,2H); 9,45 (d,1H).
I.R. : 3300, 3200, 1670, 1610, 1510, 1480, 1380, 1280, 1240, 1180, 1130, 850, 790, 700 cm⁻¹

### Exemple 5 : (3R)4-amino-N-[9-(4-amino-3,5-dichlorobenzamido)-4-oxo-1-phényl-3,4,6,7-tétrahydro-[1,4]diazépino[6,7,1-hi]indol-3-yl]-3,5-dichloro-benzamide

[(I_{eb}) ;A = 4-amino-3,5-dichloro-phényl ; B = -NR₂R₃; R₂ = R₄ =-CO-A ; R₃ = H]

Les fractions impures obtenues au traitement chromatographique de l'exemple 4 précédent sont réunies et évaporées sous vide. Le résidu est repris et traité par chromatographie rapide sur colonne de silice en éluant par du dichlorométhane progressivement enrichi en acétone. On élue le produit purifié avec le mélange de proportions 80-20 (v/v). Poids après évaporation: 0,20 g - Poudre beige-blanche - F = >220 °C
Analyse conforme pour C₃₁H₂₂N₆O₃Cl₄, (0.5 CH₃-CO-CH₃) - C.C.M. : S.A3 ; 0,60 R.M.N.¹Hδ(ppm) :3,00-3,15 (m,1H); 3,15-3,30 (m,1H); 3,95 (q,1H); 4,50-4,60 (m,1H); 4,80 (s,2H); 4,90 (s,2H); 5,40 (d,1H); 7,20-7,45 (m,6H); 7,70 (s,2H); 7,80 (s,2H); 7,85 (d,1H); 7,95 (s,1H); 8,70 (s,1H)
I.R. : 3250, 1610, 1530, 1480, 1370, 1260, 880, 780 cm⁻¹

### Exemple 6 : (3R)-2-acétylamino-N-(9-amino-4-oxo-1-phényl-3,4,6,7-tétrahydro-[1,4]diazépino[6,7,1-hi]indol-3-yl)-benzamide

[(Iₑ) ; A = 2-acetylaminophényl; B = -NR₂R₃; R₂ = R₃ = H]

Dans un réacteur de 100 ml protégé de l'humidité et sous atmosphère d'azote on dissout 1,23 g (6,84 mmol) d'acide 2-acétylamino-benzoïque dans 40 ml de tétrahydrofurane (THF) anhydre 0,76 g (7,5 mmol) de 4-méthylmorpholine.

La solution est agitée durant 5 minutes puis refroidie à - 20 °C, on ajoute alors 0,93 g (6, 84 mmol) de chloroformate d'isobutyle, puis maintient la suspension blanche sous agitation à - 20 °C durant 30 minutes. On ajoute ensuite, à cette même température, 2,00 g (6,84 mmol) de l'amine intermédiaire 2 en solution dans 11 ml de THF.

La suspension est maintenue une heure sous agitation à - 20 °C, puis 2 heures à 0 °C.

Après quoi l'insoluble est filtré, le filtrat est concentré sous vide et le résidu purifié par chromatographie rapide sur colonne de silice. On élue par le dichlorométhane et récupère 1,50 g de produit purifié amorphe qui sont cristallisés dans 100 ml d'éther éthylique. Le précipité est filtré puis séché. Poids : 1,20 g - Rdt = 39 % - Poudre jaune - F = 275 °C Analyse conforme pour C₂₆H₂₃N₅O₃ - C.C.M. : S.A6; 0,40
R.M.N.¹Hδ(ppm) : 2,20 (s,3H); 3,00-3,10 (m,1H); 3,20-3,30 (m,1H); 3,70-3,80 (m,2H éch.); 3,85-4,00 (m,1H); 4,55-4,70 (m,1H); 5,55 (d,1H); 6,50 (s,1H); 6,85 (s,1H); 7,30-7,60 (m,7H); 7,85(d,1H); 8,10 (d,1H); 8,60 (d,1H), 11,00 (m,1H)
I.R. : 3300; 1690, 1640, 1500, 1440, 1370, 1300, 1270, 1240, 1190, 1100, 960, 850, 750, 700 cm⁻¹

### Exemple 7 : (3R)N-(9-amino-4-oxo-1-phényl-3,4,6,7-tétrahydro-[1,4]diazépino[6,7,1-hi]indol-3-yl)-2-méthoxy-benzamide

[(Iₑ) ; A = 2-méthoxyphényl; B = -NR₂R₃ ; R₂ = R₃ = H]

Le composé est préparé selon le mode opératoire de l'exemple 2, à partir de l'amine intermédiaire 2 et de l'acide 2-méthoxybenzoique. Le produit est purifié par chromatographie rapide sur colonne de silice en éluant par le mélange S.A8. Après évaporation des solvants le produit purifié est concrétisé dans l'éther éthylique.
- Rdt = 65,5 % - Poudre jaune- F = 234 °C - [α]_{D} = + 39,5 ° (c = 1, CH₂ Cl₂)

Analyse conforme pour C₂₅H₂₂O₃N₄ - C.C.M. : S.A3; 0,60
R.M.N.¹Hδ(ppm) :3,00-3,10 (m,1H); 3,15-3,30 (m,1H); 3,75 (m,2H); 3,95 (q,1H); 4,05 (s,3H); 4,55-4,70 (m,1H); 5,65 (d,1H); 6,45 (s,1H);6,85 (s,1H); 7,00 (d,1H); 7,10 (t,1H); 7,35 (t,2H); 7,40-7,55 (m,2H); 7,60 (d,2H); 8,25 (d,1H); 9,85 (d,1H).
I.R. : 3300, 1670, 1640, 1600, 1500, 1480, 1470, 1240, 1160, 1020, 750, 700 cm⁻¹

### Exemple 8 : (3R)4-amino-N-(9-amino-4-oxo-1-phényl-3,4,6,7-tétrahydro-[1,4]diazépino[6,7,1-hi]indol-3-yl)-5-chloro-2-méthoxy-benzamide

[(Iₑ) ; A = 4-amino-5-chloro-2-méthoxyphényl; B = NR₂R₃ ; R₂ = R₃ = H]

Le composé est préparé selon le mode opératoire de l'exemple 2, à partir de l'amine intermédiaire 2 et de l'acide 4-amino-5-chloro-2-méthoxybenzoïque. Le produit est purifié par chromatographie rapide sur colonne de silice en éluant par le mélange dichlorométhane progressivement enrichi en méthanol.
- Rdt = 38 % -Poudre jaune - F = 198-200 °C - [α]_{D} = + 36,5 ° (c = 0,5, MeOH)

Analyse conforme pour C₂₅H₂₂ClN₅O₃- C.C.M. : S.A3; 0,50
R.M.N.¹Hδ(ppm) :2,95-3,10(s,1H); 3,15-3,3 (m,1H); 3,75 (s,2H); 3,95 (s,3H); 3,85-4,00 (m,1H); 4,55 (s,2H); 4,55-4,65 (m,1H); 5,65 (d,1H); 6,35 (s,1H); 6,55 (d,1H); 6,80 (s,1H); 7,30-7,45 (m,3H); 7,55 (d,2H); 8,15 (s,1H); 9,65 (d,1H).
I.R. : 3300, 1660, 1620, 1580, 1470, 1370,1300, 1240, 1160, 1110, 1040, 980, 700 cm⁻¹

### Exemple 9 : (3R)N-(9-amino-4-oxo-1-phényl-3,4,6,7-tétrahydro-[1,4]diazépino[6,7,1-hi]indol-3-yl)-3-cyclopentyloxy-4-méthoxy-benzamide

[(Iₑ) ; A = 3-cyclopentyloxy-4-méthoxyphényl; B = NR₂R₃ ; R₂ = R₃ = H]

Composé préparé selon le mode opératoire de l'exemple 2, à partir de l'amine intermédiaire 2 et d'acide 3-cyclopentyloxy-4-méthoxybenzoïque (préparé selon J. Med. Chem. **1994.,** *37*, 1696-1703). Après purification par chromatographie rapide sur colonne de silice en éluant par l'acétate d'éthyle, le produit est finalement concrétisé par dissolution dans l'acétate d'éthyle puis précipitation par l'hexane.
- Rdt = 63 % - Poudre jaune- F = 138-146 °C - [α]_{D} = + 48 ° (c = 1, CH₂ Cl₂)

Analyse conforme pour C₃₀H₃₀N₄O₄- C.C.M. : S.B; 0,40
R.M.N.¹Hδ(ppm) : 1,55-1,70 (m,2H); 1,75-2,10 (m,6H); 2,95-3,10 (m,1H); 3,20-3,35 (m,1H); 3,80 (s,2H); 3,90 (s,3H); 3,904,00 (m,1H); 4,55-4,70 (m,1H); 4,85-4,95 (m,1H); 5,55-5,65 (d,1H); 6,45 (d,1H); 6,80-7,00 (m,2H); 7,30-7,65 (m,7H), 7,90-8,00 (d,1H)
I.R. : 3320, 2920, 1650, 1480, 1370, 1260, 1160, 1020, 760, 700, 520 cm⁻¹

### Exemple 10 : (3R)Pyridine-2-acide carboxylique (9-amino-4-oxo-1-phényl-3,4,6,7-tétrahydro-[1,4]diazépino[6,7,1-hi]indol-3-yl)-amide

[(Iₑ) ; A = 2-pyridyl ; B = -NR₂R₃; R₂ = R₃ = H]

Préparation selon le mode opératoire de l'exemple 2, à partir de l'amine intermédiaire 2 et de l'acide picolinique (ou pyridine-2-carboxylique). Le produit est purifié par recristallisation de l'isopropanol.
- Rdt = 50 % - Poudre jaune - F = 266-267 °C - [α]_{D} = + 67 ° (c = 1, CH₂ Cl₂)

Analyse conforme pour C₂₃H₁₉N₅O₂- C.C.M. : S.A2; 0,45
R.M.N.¹Hδ(ppm) : 2,95-3,10 (m,1H); 3,15-3,35 (m,1H); 3,70-3,85 (s,2H); 3,85-4,00 (m,1H); 4,55-4,70 (m, 1H); 5,55-5,70 (d,1H); 6,45 (s,1H); 6,85 (s, 1H); 7,30-7,70 (m,6H); 7,80-7,90 (m,1H); 8,15-8,30 (m,1H); 8,60-8,70 (m,1H); 9,60-9,80 (d,1H)
I.R. : 3320, 1660, 1500, 1370, 1270, 1230, 1170, 995, 830, 690 cm⁻¹

### Exemple 11 : (3R)N-(9-amino-4-oxo-1-phényl-3,4,6,7-tétrahydro-[1,4]diazépino[6,7,1-hi]indol-3-yl)-nicotinamide

[(Iₑ) ; A = 3-pyridyl ; B = -NR₂R₃ ; R₂ = R₃ = H]

Préparation selon le mode opératoire de l'exemple 10 précédent avec l'amine intermédiaire 2 et de l'acide nicotinique (ou pyridine-3-carboxylique). Après purification par chromatographie en éluant par le mélange S.A9, le produit est cristallisé dans le méthanol.
- Rdt = 33 % -Poudre jaune- F = > 250 °C

Analyse conforme pour C₂₃H₁₉N₅O₂ (0.4 MeOH) - C.C.M. : S.A1; 0,50
R.M.N.¹Hδ(ppm) : 2,95-3,10 (m,1H); 3,20-3,30 (m,1H); 3,80-3,90 (m,1H); 4,40-4,50 (m,1H); 5,30 (s,2H); 5,55 (d,1H); 6,35 (s,1H); 6,90 (s,1H); 7,40-7,60 (m,6H); 8,35-8,40 (m,1H); 8,75 (d,1H); 9,15 (s,1H); 9,90 (d,1H)
I.R. : 3300, 3200, 1660, 1580, 1460, 1380, 1265, 1235, 1165, 1020, 840, 695 cm⁻¹

### Exemple 12 A: (3R)N-(9-amino-4-oxo-1-phényl-3,4,6,7-tétrahydro-[1,4]diazépino[6,7,1-hi]indol-3-yl)-isonicotinamide

[(Iₑ) ; A = 4-pyridyl ; B = -NR₂R₃ ; R₂ = R₃ = H]

Préparation selon le mode opératoire de l'exemple 10 avec l'amine intermédiaire 2 et l'acide isocotinique (ou pyridine-4-carboxylique). Après purification par chromatographie en éluant par le mélange S.A10, le produit est concrétisé dans l'éther diéthylique.
- Rdt = 41 % - Poudre jaune - F = > 280 °C - [α]_{D} = + 60,8 ° (c = 1, MeOH)

Analyse conforme pour C₂₃H₁₉N₅O₂ - C.C.M. : S.A1; 0,50
R.M.N.¹Hδ(ppm) : 2,95-3,08 (m,1H); 3,20-3,30 (m,1H); 3,80-3,90 (m,1H); 4,35-4,48 (m,1H); 5,25 (s,2H éch.); 5,45 (d,1H); 6,00 (s,1H); 6,90 (s,1H); 7,40-7,60 (m,5H); 7,95 (d,2H); 8,80 (d,2H); 9,90 (d,1H) -
I.R. : 3300, 3200, 1660, 1480, 1380, 1240, 1170, 1060, 840, 695, 780, 750, 690 cm⁻¹
- sulfate : dans un réacteur on introduit sous agitation 1,00g (2,5 mmol) du composé obtenu ci-dessus dans 20 ml d'éthanol anhydre. A la suspension on ajoute goutte à goutte 0,50 ml d'acide sulfurique concentré (d = 1,84) . La température de la suspension s'élève et le milieu se colore. On ajoute à nouveau 0,50 ml d'acide et laisse agiter une heure. L'insoluble est filtré, lavé à l'éthanol puis séché. Poids : 1,00 g - F = > 230 °C -

[α]_{D} = - 37,0° (c = 1, H₂O) - C.C.M. : S.A10; 0,50
Analyse conforme pour C₂₃H₁₉N₅O₂, 2 H₂SO₄, (3H₂O)

### Exemple 12 B: (3R)N-(9-acétamido-4-oxo-1-phényl-3,4,6,7-tétrahydro-[1,4]diazépino[6,7,1-hi]indol-3-yl)-isonicotinamide

[(I_{eb}); A = 4-pyridyl; B = -NR₂R₃ ; R₂ = R₄ = CH₃-CO-; R₃ = H]

Dans un réacteur anhydre on dissout 1,50 g (3,7 mmol) du composé de l'exemple 12A sous sa forme de base dans 15,1 ml de pyridine anhydre. On ajoute à la solution 7,3 ml soit 7,85 g (77 mmol) d'anhydride acétique puis la solution est abandonnée sous agitation à 20-25°C durant 16 heures. On y ajoute 70 ml d'eau et poursuit l'agitation durant 4 heures, Le mélange est alors extrait par 3 fois 75 ml d'acétate d'éthyle, les phases organiques réunies sont lavées par 2 fois 75 ml de solution saturée en bicarbonate de sodium puis séchées. Les solvants sont éliminés par distillation sous vide et sur bain marie puis le résidu purifié par chromatographie rapide sur colonne de silice. L'élution par un mélange dichlorométhane-méthanol 90 - 10 (v/v) permet de récupérer le produit purifié à l'état amorphe.
Poids : 1,20 g - Rdt = 71% Poudre jaune - F = 204 - 205°C
Analyse conforme pour C₂₅H₂₁N₅O₃ (0.75 H₂O) - C.C.M. : S.A10; 0,50
R.M.N.¹Hδ(ppm) : 2,10 (s,3H); 3,05-3,20 (m, 1H); 3,25-3,40 (m,1H); 3,95 (q,1H); 4,65 (t,1H); 5,45 (d,1H); 7,05 (s,1H); 7,30-7,50 (m,5H); 7,80 (d,2H); 8,00 (s,1H); 8,10 (s,1H); 8,20 (d,1H); 8,80 (d,2H)
I.R. : 3200, 1650, 1330, 1370, 1280, 1160, 1060, 840,, 700 cm⁻¹

### Exemple 13 : (3R)3-t.butyloxycarbonylamino-N-(9-amino-4-oxo-1-phényl-3,4,6,7-tétrahydro-[1,4]diazépino[6,7,1-hi]indol-3-yl)-isonicotinamide

[(Iₑ) ; A = 2-t.butyloxycarbonylamino-4-pyridyl ; B = -NR₂R₃; R₂ = R₃ = H]

Dans un réacteur protégé de l'humidité et sous atmosphère d'azote on dissout dans 10 ml de tétrahydrofurane anhydre 0,50 g (1,7 mmol) de l'aminé intermédiaire 2 et 0,41 g (1,7 mmol) d'acide 3-t-butyloxycarbonylamino-pyridine-4-carboxylique préparé par réaction dans le dioxane de l'acide 3-amino-isonicotinique avec le di t.butyl dicarbonate.

On ajoute ensuite sous agitation à 20-25 °C 0,96 g (2,05 mmol) de PyBrop (hexafluorophosphate de bromo-tris-pyrrolidino-phosphonium) et 0,52 g (5,1 mmol) de triéthylamine.

Le mélange est agité 16 heures à 20-25 °C, l'insoluble est écarté par filtration et le filtrat concentré sous vide sur bain marie.

Le résidu est purifié par chromatographie rapide sur colonne de silice. L'élution par le mélange S.A8 permet d'obtenir 0,55 g de produit purifié.
Rdt = 63 % - Poudre cristallisée jaune -F = 221-225 °C - [α]_{D} = - 24,5 ° (c = 0,5, CH₂Cl₂)
Analyse conforme pour C₂₈H₂₈N₆O₄, (0.6 H₂O) - C.C.M. : S.A1; 0,70
R.M.N.¹Hδ(ppm): 1,4 (s,9H); 2,95-3,10 (m,1H); 3,30-3,45 (m,1H); 3,85 (q,1H); 4,40 (t,1H); 5,30 (s,2H); 5,40 (d,1H); 6,35 (d,1H); 6,90 (d,1H); 7,35-7,60 (m,5H); 7,90 -(d,1H); 8,40 (d,1H); 9,45 (s,1H); 9,90 (s,1H); 10,05 (d,1H).
I.R. : 3350, 1720, 1650, 1560, 1510, 1410, 1370, 1240, 1150, 1050, 1020, 700 cm⁻¹

### Exemple 14 : (3R)3-amino-N-(9-amino-4-oxo-1-phényl-3,4,6,7-tétrahydro-[1,4]diazépino[6,7,1-hi]indol-3-yl)-isonicotinamide

[(Iₑ) ; A = 2-amino-4-pyridyl ; B = -NR₂R₃ ; R₂ = R₃ = H]

Dans un réacteur on dissout 0,55 g (1,07 mmol) du (3R)3-t.butyloxycarbonylamino-N-(9-amino-4-oxo-1-phényl-3,4,6,7-tétrahydro-[1,4]diazépino[6,7,1-hi]indol-3-yl)-isonicotinamide obtenus à l'exemple précédent dans 20,0 ml de dichlorométhane anhydre. On ajoute goutte à goutte sous agitation à 20-25 °C 4,27 g (37,5 mmol) d'acide trifluoroacétique pur. (d = 1,480). Après 30 minutes d'agitation à 20-25 °C on concentre par distillation sous vide et sur bain maris. le résidu est dissous dans 25 ml d'acétate d'éthyle, la solution est lavée par 2 fois 10 ml de solution saturée en bicarbonate de sodium puis desséchée. Le solvant est éliminé par distillation sous vide et le résidu purifié par chromatographie rapide sur silice. L'élution par le dichlorométhane progressivement enrichi en méthanol permet d'obtenir 0,17 g de produit purifié conforme.
- Rdt = 38 % -Poudre cristallisée jaune - F = 215-230 °C - [α]_{D} = - 4,35 ° (c = 0,5, CH₂Cl₂)

Analyse conforme pour C₂₃H₂₀N₆O₂, (0.3 CH₂Cl₂), (0.8 H₂ O) - C.C.M. : S.A1; 0,55
R.M.N.¹Hδ(ppm): 2,95-3,10 (m,1H);3,20-3,35 (m,1H); 3,30-4,10 (m,2H); 3,85-4,00 (q,1H); 4,50-4,70 (t,1H); 5,55 (d,1H); 6,45 (s,1H); 6,85 (s,1H); 7,30-7,50 (m,4H); 7,50-7,65 (m,2H); 7,95 (d,1H); 8,05-8,25 (m,2H).
I.R. : 3300, 1640, 1580, 1480, 1370, 1230, 1050, 780, 700 cm⁻¹

### Exemple 15 : (3R)3-acétylamino-N-(9-acétylamino-4-oxo-1-phényl-3,4,6,7-tét rahydro-[1,4]diazépino[6,7,1-hi]indol-3-yl)-isonicotinamide

[(I_{eb}) ; A = 2-acétylamino-4-pyridyl ; B = -NR₂R₃; R₂ = R₄ = -CO-CH₃ ; R₃ = H]

Dans un ballon protégé de l'humidité on introduit 0,130 g (0,315 mmol) de (3R)3-amino-N-(9-amino-4-oxo-1-phényl-3,4,6,7-tétrahydro-[1,4]diazépino[6,7,1-hi]indol-3-yl)-isonicotinamide obtenu à l'exemple précédent, 1,3 ml de pyridine anhydre et 0,69 g (6,75 mmol) d'anhydride acétique.

Le mélange est agité 16 heures à 20-25 °C puis on ajoute 6,5 ml d'eau et poursuit l'agitation durant 5 heures, on extrait par deux fois 15 ml d'acétate d'éthyle, les phases organiques réunies sont lavées par une solution saturée en bicarbonate de sodium puis séchées, L'acétate d'éthyle est éliminé par distillation puis le résidu est purifié par chromatographie rapide sur silice.

L'élution par le dichlorométhane progressivement enrichi en méthanol permet d'obtenir 0,050 g de produit purifié.
- Rdt = 32 % - Poudre amorphe jaune pâle

Analyse conforme pour C₂₇H₂₄O₄N₆ - C.C.M. : S.A1; 0,52
R.M.N.¹Hδ(ppm) : 2,10 (s,3H); 2,15 (s,3H); 3,20-3,05 (m,1H); 3,25-3,45 (m,1H); 3,95 (q, 1H); 4,60 (t,1H); 5,50 d,1H); 7,15 (s,1H); 7,35 (t,2H); 7,40 (t,1H); 7,45 (d,2H); 7,55 (d,1H); 7,90 (s,1H); 8,30 (d,1H); 8,45 d,1H); 8,55 (s,1H);9,75 (s,1H); 10,45 (s,1H).

### Exemple 16 : (3R)N-(9-amino-4-oxo-1-phényl-3,4,6,7-tétrahydro-[1,4]diazépino[6,7,1-hi]indol-3-yl)-3,5-dichloro-isonicotinamide

[(Iₑ) ; A = 3,5-dichloro-4-pyridyl ; B = -NR₂R₃ ; R₂ = R₃ = H]

Le composé est préparé selon le mode opératoire de l'exemple 2, à partir de l'amine intermédiaire 2 et de l'acide 3,5-dichloropyridine-4-carboxylique.

Le produit est purifié par chromatographie, l'élution étant réalisée par le mélange S.A8. Le produit est finalement cristallisé dans un mélange acétate d'éthyle-hexane.
- Rdt = 32 % - Poudre jaune - F = 165-166 °C - [α]_{D} = + 175 ° (c = 1, CH₂Cl₂) Analyse conforme pour C₂₃H₁₇Cl₂N₅O₂, (0.4 AcOEt) - C.C.M. : S.A3; 0,60

R.M.N.¹Hδ(ppm) : 3,00-3,15 (m,1H); 3,25-3,40 (m,1H); 3,70-3,85 (s,2H); 3,85-4,00 (m,1H); 4,55-4,70 (m, 1H); 5,60-5,70 (d,1H); 6,50(s,1H); 6,85 (s,1H); 7,35-7,65 (m,5H); 7,80-7,95 (d,1H); 8,60 (s,2H).
I.R. : 3320, 1660, 1560, 1370, 1230, 1200, 1090, 1030, 820, 700 cm⁻¹

### Exemple 17 : (3R)pyrazine-2-carboxylique acide (9-amino-4-oxo-1-phényl-3,4,6,7-tétrahydro-[1,4]diazépino[6,7,1-hi]indol-3-yl)-amide

[(Iₑ) ; A = 2-pyrazyl ; B = -NR₂R₃ ; R₂ = R₃ = H]

Composé préparé selon le mode opératoire de l'exemple 2 avec l'amine intermédiaire 2 et l'acide pyrazine-2-carboxylique. Le produit est purifié par chromatographie rapide puis cristallisation dans l'hexane.
- Rdt = 59 % - Poudre jaune - F = 264 °C - [α]_{D} = + 67 ° (c = 1, CH₂Cl₂)

Analyse conforme pour C₂₂H₁₈N₆O₂ - C.C.M. : S.A2; 0,40
R.M.N.¹Hδ(ppm) : 2,95-3,10 (m,1H); 3,20-3,35 (m,1H); 3,80-3,95 (m,1H); 4,35-4,50 (m,1H); 5,30-5,40 (m,3H); 6,40 (d,1H); 6,85 (d,1H); 7,40-7,55 (m,5H); 8,80-9,00 (m,2H); 9,30 (s,1H); 9,40-9,50 (d,1H)
I.R. : 3350, 2900, 1670, 1500, 1370, 1170, 1040, 850, 690, 530 cm⁻¹

### Exemple 18 : (3R)isoquinoline-3-carboxylique acide (9-amino-4-oxo-1-phényl-3,4,6,7-tétrahydro-[1,4]diazépino[6,7,1-hi]indol-3-yl)-amide

[(Iₑ) ; A = 3-isoquinolyl ; B = -NR₂R₃ ; R₂ = R₃ = H]

Composé préparé selon le mode opératoire de l'exemple 2 avec l'amine intermédiaire 2 et l'acide isoquinoline-3-carboxylique. Le produit purifié est obtenu par cristallisation et chromatographie rapide sur silice avec le mélange d'élution S.A8.
- Rdt = 55 % - Poudre jaune - F = > 282 °C - [α]_{D} = + 16 ° (c = 1, CH₂Cl₂)

Analyse conforme pour C₂₇H₂₁N₅O₂ - C.C.M. : S.A3; 0,80
R.M.N.¹Hδ(ppm) : 3,00-3,10 (m, 1H); 3,20-3,30 (m,1H); 3,80-3,90 (m,1H); 4,40-4,50 (m,1H); 5,30 (s,2H); 5,40 (d,1H); 6,40 (s,1H); 6,90 (s,1H); 7,40-7,55 (m,5H); 7,20-7,95 (m,2H); 8,20-8,35 (m,2H); 8,65 (s,1H); 9,5 (s,1H), 9,65 (s,1H)
I.R. : 3300, 3200, 1670, 1500, 1380, 1300, 1270, 1230, 1160, 940, 935, 850, 740, 700 cm⁻¹

### Exemple 19 : (3R)quinoline-3-carboxylique acide (9-amino-4-oxo-1-phényl-3,4,6,7-tétrahydro-[1,4]diazépino[6,7,1-hi]indol-3-yl)-amide

[(Iₑ); A = 3-quinolyl; B = -NR₂R₃; R₂ = R₃ = H]

Composé préparé selon le mode opératoire de l'exemple 2 avec l'amine intermédiaire 2 et l'acide quinoline-3-carboxylique. Le produit est purifié par chromatographie rapide sur silice en éluant par le dichlorométhane progressivement enrichi en méthanol.
- Rdt = 44 % - Poudre jaune - F = 277 °C - [α]_{D} = + 75,5 ° (c = 0,5, CH₂Cl₂) Analyse conforme pour C₂₇H₂₁N₅O₂

R.M.N.¹Hδ(ppm): 3,00-3,15 (m,1H); 3,20-3,40 (m,1H); 4,3 (s,2H); 3,95 (q,1H);4,60-4,70 (m,1H); 5,65 (d,1H); 6,50 (d,1H); 6,85 (m,1H); 7,55-7,30 (m,3H); 7,60 (d,2H); 7,65 (m,1H); 7,85 (m,1H); 7,95 (d,1H); 8,15 (d,1H); 8,25 (d,1H); 8,75 (d,1H); 9,45 (d,1H).
I.R. : 3300, 3200, 1650, 1620, 1500, 1480, 1240, 1220, 790, 760, 690 cm⁻¹

### Exemple 20 : (3R)4,7-diméthyl-pyrazolo[5,1-c][1,2,4]triazine-3-carboxylique acide (9-amino-4-oxo-1-phényl-3,4,6,7-tétrahydro-[1,4]diazépino[6,7,1-hi]indol-3-yl)-amide

[(Iₑ) ; A = 4,7-diméthyl-pyrazolo[5,1-c][1,2,4]-3-triazinyl; B = -NR₂R₃ ; R₂ = R₃ = H]

Composé préparé selon le mode opératoire de l'exemple 2 et à partir de l'intermédiaire 2 de l'acide 4,7-diméthyl-pyrazolo[5,1-*c*[1,2,4]triazine-3-carboxylique. Le produit est purifié par chromatographie rapide sur silice en éluant par le dichlorométhane progressivement enrichi en méthanol.
- Rdt = 40 % - Poudre jaune orangée- F = 168-170 °C -

Analyse conforme pour C₂₅H₂₂N₈O₂, (0.5 H₂O) - C.C.M. : S.A3; 0,60
R.M.N.¹Hδ(ppm):2,65 (s,3H); 3,00-3,15 (m,1H); 3,25-3,35 (m,1H); 3,30 (s,3H), 3,70-3,80 (m,2H); 3,95 (q,1H); 4,55-4,75 (m,1H); 5,65 (d,1H); 6,50 (d,1H); 6,85(d,1H); 7,10 (s,1H); 7,35 -7,45 (m,3H); 7,60 (d,2H); 9,85 (d,1H).
I.R. : 3300, 1660, 1560, 1480, 1370, 1300, 1240, 1160, 850, 780, 700 cm⁻¹

### Exemple 21 : (3R)4-amino-3,5-dichloro-N-(9-diméthylamino-4-oxo-1-phényl-3,4,6,7-tétrahydro-[1,4]diazépino[6,7,1-hi]indol-3-yl)-benzamide

[(I_{ed}) ; A = 4-amino-3,5-dichlorophényl ; B = -NR₂R₃ ; R₂ = R₃ = CH₃]

Dans un réacteur de 250 ml on dissout 2,00 g (4,2 mmole) de (3R)4-amino-N-(9-amino-4-oxo-1-phényl-3,4,6,7-tétrahydro-[1,4]diazépino[6,7,1-hi]indol-3-yl)-3,5-dichloro-benzamide (produit de l'exemple 4) dans 100 ml d'acétonitrile.

Sous agitation, à 20-25 °C on ajoute 3,43 ml de solution de formaldehyde à 37 % (42 mmol) puis 0,80 g (12,5 mmol) de cyanoborohydrure de sodium et 0,50 ml d'acide acétique pur. On agite deux heures à température ambiante puis ajoute à nouveau 0,50 ml d'acide acétique, après quoi on agite 15 minutes puis précipite le mélange dans 350 ml d'éther éthylique.

Le précipité blanc qui se forme est filtré et écarté, le filtrat est extrait par deux fois 100 ml de solution NaOH N. La phase éthérée est lavée par une solution saturée de NaCl puis déshydratée. L'éther éthylique est éliminé par distillation et le résidu purifié par chromatographie rapide sur silice. L'élution par le dichlorométhane progressivement enrichi en acétone conduit à récupérer 0,80 g de produit purifié qui sont concrétisés dans un mélange éther éthylique/heptane. Le produit est filtré puis séché. Poids 0,75 g.
- Rdt = 35 % - Poudre jaune orangée- F = 174-176 °C -

Analyse conforme pour C₂₆H₂₃Cl₂N₅O₂, (0.3 H₂O) - C.C.M. : S.A3; 0,75
R.M.N.¹Hδ(ppm) : 2,90 (s,6H); 3,00-3,15 (m,1H); 3,25-3,40 (m,1H); 3,95 (q,1H); 4,65 (t,1H); 4,85 (s,2H); 5,60 (d,1H); 6,45 (d,1H); 6,95 (s,1H); 7,35 (t,2H); 7,40-7,50 (m,1H); 7,60 (d,2H); 7,80-7,95 (m,3H).
I.R. 3300, 1650, 1610, 1470, 1370, 1270, 1220, 1120, 780, 700 cm⁻¹

### Exemple 22 : (3R)4-amino-3,5-dichloro-N-(4-oxo-1-phényl-9-pyrrolidin-1-yl-3,4,6,7-tétrahydro-[1,4]diazépino[6,7,1-hi]indol-3-yl)-benzamide

[(Iₑₑ) ; A = 4-amino-3,5-dichloro-phényl ; B = -NR₂R₃ ; R₂ -R₃ = -(CH₂)₄-]

Dans un ballon de 250ml on dissout 3,00g (6,0 mmol) de (3R)4-amino-N-(9-amino-4-oxo-1-phényl-3,4,6,7-tétrahydro-[1,4]diazépino[6,7,1-hi]indol-3-yl)-3,5-dichloro-benzamide (produit de l'exemple 4) dans 100 ml d'acétonitrile. On ajoute 1,48g (6,9 mmol) de 1,4-dibromobutane et porte au reflux sous agitation durant 16 heures. L'acétonitrile est éliminé par distillation sous vide, le résidu repris par 250 ml d'eau et alcalinisé par de la lessive de soude. Le mélange est extrait par 3 fois 100 ml de dichlorométhane, les phases organiques réunies sont lavées puis séchées et le solvant est éliminé par distillation. Le résidu est purifié par chromatographie rapide sur colonne de silice en éluant par de dichlorométhane progressivement enrichi en acétone. Poids : 0,75g. Rdt = 7,5%.

Analyse conforme pour C₂₈H₂₅Cl₂N₅O₂ - C.C.M. : S.A6; 0,85
R.M.N.¹Hδ(ppm) : 1,90-2,05 (m,4H); 3,00-3,10 (m,1H); 3,15-3,25 (m,4H);3,25-3,40 (m,1H); 3,95 (q,1H); 4,60 (t,1H); 4,80 (s,2H); 5,50 (d,1H); 6,25 (s,1H); 6,75 (s,1H); 7,30-7,50 (m,3H); 7,60 (d,2H); 7,80-7,95 (m,3H).
I.R. : 3300, 1650, 1610, 1470, 1380, 1270, 890, 780, 700 cm⁻¹

### Exemple 23 : (3R)4-amino-3,5-dichloro-N-(4-oxo-1-phényl-9-morpholin-1-yl-3,4,6,7-tétrahydro-[1,4]diazépino[6,7,1-hi]indol-3-yl)-benzamide

[(Iₑₑ) ; A = 4-amino-3,5-dichloro-phényl ; B = -NR₂R₃ ; R₂ -R₃ = -(CH₂)₂-O-(CH₂)₂-]

Le composé est préparé selon le mode opératoire de l'exemple 22 précédent à partir de 1,0g (2,1 mmol) de (3R)4-amino-N-(9-amino-4-oxo-1-phényl-3,4,6,7-tétrahydro-[1,4]diazépino[6,7,1-hi]indol-3-yl)-3,5-dichloro-benzamide (produit de l'exemple 4) dans 35 ml d'acétonitrile et 0,67 g (3,1 mmol) de 2-dibromoéthyl éther. le produit est purifié par chromatographie rapide. C.C.M : SA6; 0,55.

R.M.N.¹Hδ(ppm): 2,90-3,10 (m,5H); 3;20-3,35 (m,1H); 3,70-3,80 (m,4H); 3,90 (q,1H); 4,60 (t,1H); 4,75 (s,2H); 5,50 (d,1H); 6,60 (s,1H); 7,05 (s,1H); 7,30-7,45 (m,3H); 7,50 (d,2H); 7,75 (d ,1H); 7,80 (s,2H).

### Exemple 24 : (3R)4-amino-3,5-dichloro-N-(9-guanidino-4-oxo-1-phényl-3,4,6,7-tétrahydro-[1,4]diazépino[6,7,1-hi]indol-3-yl)-benzamide

[(Iₑₐ) ; A = 4-amino-3,5-dichloro-phényl ; B = -NR₂R₃ ; R₂ = -C(NH) NH₂R₃ = H]

Dans un réacteur de 250 ml on introduit 2,02 g (4,2 mmol) de (3R)4-amino-N-(9-amino-4-oxo-1-phényl-3,4,6,7-tétrahydro-[1,4]diazépino[6,7,1-hi]indol-3-yl)-3,5-dichloro-benzamide en solution dans 50 ml de dichlorométhane. On ajoute 5 ml d'éther chlorhydrique 4N puis évapore les solvants sous vide.

Le résidu est repris sous agitation par 150 ml d'acétonitrile et sous agitation à 20-25 °C on ajoute 0,192 g (4,6 mmol) de cyanamide. Sous agitation la suspension est chauffée et maintenue au reflux durant 41 heures. Après refroidissement à environ 10 °C l'insoluble est filtré, repris par 25 ml d'eau.Le mélange est alcalinisé par de la lessive de soude 10 N et extrait par 2 fois 50 ml de dichlorométhane. Les phases organiques réunies sont lavées, séchées puis le solvant est éliminé par distillation. Le produit brut (1,0 g) est purifié par chromatographie rapide sur silice. L'élution par un mélange de dichlorométhane et de méthanol ammoniacal à 10 % en proportion 50-50 permet de purifier le produit attendu. Poids = 0,27 g
- Rdt = 13 % -Poudre jaune pâle - F = > 220 °C

Analyse conforme pour C₂₅H₂₁Cl₂N₇O₂, (4 H₂O) - C.C.M. : S.C; 0,20
R.M.N.¹Hδ(ppm) : 3,00-3,15 (m,1H) ; 3,20-3,35 (m,1H); 3,90 (q,1H); 4,45 (t,1H); 5,45 (d,1H); 6,80 (s,1H); 7,30 (s,1H); 7,40 (t,2H); 7,45-7,60 (m,3H); 8,00 (s,2H) 8,65 (s,2H); 9,55 (d,1H).

### Exemple 25 : (3R)-N-(9-Acétylamino-4-oxo-1-phényl-3,4,6,7-tétrahydro-[1,4]diazépino[6,7,1-hi]indol-3-yl)-4-amino-3,5-dichloro-benzamide

[(I_{eb}) ; A = 4-amino-3,5-dichloro-phényl ; B = -NR₂R₃ ; R₂ = R₄ = -C(O)CH₃; R₃ = H]

Dans un ballon de 25 ml on introduit 2,50 g (3,12 mmol) de (3R)4-amino-N-(9-amino-4-oxo-1-phényl-3,4,6,7-tétrahydro-[1,4]diazépino[6,7,1-hi]indol-3-yl)-3,5-dichloro-benzamide en solution dans 12,85 ml de pyridine anhydre puis on ajoute 6,70 g soit 6,2 ml (6,56 mmol) d'anhydride acétique. la solution est agitée durant 16 heures à 20-25°C puis on y ajoute 65 ml d'eau et laisse agiter 4 heures à température ambiante. Après quoi le mélange est extrait par 3 fois 75 ml d'acétate d'éthyle, les phases organiques réunies sont lavées par une solution saturée en bicarbonate de sodium puis séchées et évaporées sous vide. Le résidu est purifié par chromatographie rapide sur colonne de silice en éluant par du dichlorométhane progressivement enrichi en méthanol. Le résidu purifié d'un poids de 2,0 g est repris par l'acétate d'éthyle et lavé par HCl puis à l'eau pour éliminer la pyridine résiduelle.
Poids : 0,70 g - Rdt = 43% - F = 192°C -
Analyse conforme pour C₂₆H₂₁Cl₂N₅O₃, (0.6 H₂O) - C.C.M. : S.A1; 0,75
R.M.N.¹Hδ(ppm) : 2,10 (s,3H); 3,00-3,15 (m,1H); 3,20-3,35 (m,1H); 3,95 (q,1H); 4,60 (t,1H); 4,85 (s,2H); 5,35 (d,1H); 7,00 (s,1H); 7,25-7,50 (m,5H); 7,80 (s,2H); 7,85 (d,1H); 7,95 (s,1H) 8,35 (s,1H)
I.R. : 3300, 1660, 1610, 1540, 1470, 1370, 1270,780, 700 cm-1

### Exemple 26 : (3R)4-amino-3,5-dichloro-N-(9-{2-[2-(2-méthoxy-éthoxy)éthoxyl]-acétylamino}-4-oxo-1-phényl-3,4,6,7-tétrahydro-[1,4]diazépino[6,7,1-hi]indol-3-yl)-benzamide

[(I_{eb}) ; A = 4-amino-3,5-dichloro-phényl ; B = -NR₂R₃ ; R₂ = R₄ = CH₃-(O-CH₂-CH₂)₂-O-CH₂-CO; R₃ = H]

Le composé est préparé selon de mode opératoire de l'exemple 2 à partir de (3R)-4-amino-N-(9-amino-4-oxo-1-phényl-3,4,6,7-tétrahydro-[1,4]diazépino[6,7,1-hi]indol-3-yl)-3,5-dichloro-benzamide (produit de l'exemple 4) et d'acide 3,6,9-trioxodecanoïque.

Le produit est purifié par chromatographie rapide sur silice en éluant par du dichlorométhane progressivement enrichi en acétone.
- Rdt = 45 % - Poudre blanche - F = 105-107 °C -

Analyse conforme pour C₃₁H₃₁Cl₂N₅O₆, (0.55 H₂O) - C.C.M. : S.A6; 0,25
R.M.N.¹Hδ(ppm) : 3,05-3,20 (m,1H); 3,25 (s,3H); 3,30-3,40 (m,3H); 3,45-3,60 (m,2H); 3,60-3,80 (m,4H); 4,00 (q,1H); 4,10 (d,2H); 4,65 (t,1H); 4,85 (s,2H); 6,45 (d,1H); 7,20 (d,1H); 7,30-7,45 (m,2H); 7,45-7,50 (m,1H); 7,55 (dd,2H); 7,80-7,90 (m,3H); 8,10 (s,1H); 8,95 (s,1H).
I.R. : 3250, 2850, 1670, 1610, 1520, 1460, 1370, 1270, 1100, 780 cm⁻¹

### Exemple 27 : (3R)(2-{2-[3-(4-amino-3,5-dichloro-benzoylamino)-4-oxo-1-phényl-3,4,6,7-tétrahydro-[1,4]diazépino[6,7,1-hi]indol-9-ylcarbomoylméthoxy]éthoxy}-éthoxy)-acétique acide

[(I_{eb}) ; A = 4-amino-3,5-dichloro-phényl ; B = -NR₂R₃ ; R₂ = R₄ = HOOC-CH₂-(O-CH₂-CH₂)₂-O-CH₂-CO; R₃ = H]

Le composé est préparé selon l'exemple 23 précédent avec l'acide 3,6,9-trioxa-undécanedioïque. Le produit est purifié par chromatographie suivie d'une concrétisation dans l'éther diéthylique.
- Rdt = 30 % - Poudre jaune pâle - F = > 230 °C -

Analyse conforme pour C₃₂H₃₁Cl₂N₅O₈, (1 H₂O) - C.C.M. : S.C;
R.M.N.¹Hδ(ppm) : 3,05-3,20 (m,1H);3,35-3,45 (m,1H); 3,45-3,65 (m,9H); 3,70 (s,2H);3,90 (q,1H); 4,10 (s,2H); 4,40 (t,1H); 5,45 (d,1H); 6,65 (s,2H); 7,40-7,60 (m,6H); 7,95 (s,1H); 8,00 (s,2H); 9,55 (d,1H); 10,15 (s,1H);
I.R. : 3250, 1610, 1520, 1460, 1370, 1270, 1100, 780; 700 cm⁻¹

### Exemple 28 : (3R)héxadecanoique acide [3-(4-amino-3,5-dichloro-benzoylamino)-4-oxo-1-phényl-3,4,6,7-tétrahvdro-[1,4]diazépino[6,7,1-hi]indol-9-yl amide

[(I_{eb}) ; A = 4-amino-3,5-dichloro-phényl; B = -NR₂R₃ ; R₂ = R₄ = CH₃-(CH₂)₁₄-CO; R₃ = H]

Dans un ballon de 25 ml, sous atmosphère d'azote on dissout 1,00 g (2,1 mmol) de (3R)4-amino-N-(9-amino-4-oxo-1-phényl-3,4,6,7-tétrahydro-[1,4]diazépino[6,7,1-hi]indol-3-yl)-3,5-dichloro-benzamide (produit de l'exemple 4) dans 10,0 ml de pyridine anhydre. On ajoute ensuite à 20-25 °C 0,63 g (2,3 mmol) de chlorure de l'acide hexadécanoïque (ou chlorure de palmitoyle). La solution est maintenue sous agitation à 20-25 °C durant 2 h 30 puis concentré sous vide. Le résidu est repris par 50 ml de mélange dichlorométhane-éther diéthylique 1-1 (v/v). La phase organique est lavée par 3 fois 25 ml de solution HClN puis successivement 3 fois 25 ml de solution NaOH 10 % et 3 fois 25 ml d'eau, après quoi elle est séchée et les solvants éliminés par distillation.

Le résidu est concrétisé dans l'heptane, filtré puis séché. Poids : 0,90 g
- Rdt = 60 % - Poudre blanche - F = 130 °C -

Analyse conforme pour C₄₀H₄₉Cl₂N₅O₃ - C.C.M. : S.A; 0,85
R.M.N.¹Hδ(ppm) :0,85 (t,3H); 1,25 (s, 24H); 1,50-1,70 (m,2H); 2,25 (t,2H); 3,05-3,15 (m,1H); 3,20-3,35 (m,1H); 3,95 (q,1H); 4,60 (t,1H); 4,85 (s,2H); 5,35 (d,1H); 7,00 (s,1H); 7,25-7,35 (m,2H); 7,40 (d,1H); 7,45 (d,2H); 7,75-7,90 (m,3H); 7,95 (d,2H).
I.R.: 3250, 2900, 2800, 1660, 1610, 1530, 1460, 1370, 1270, 1230, 1120, 880, 780 cm⁻¹

### Exemple 29 : (3R)isoquinoline-3-carboxylique acide(9-acétylamino-4-oxo-1-phényl-3,4,6,7-tétrahydro-[1,4]diazépino[6,7,1-hi]indol-9-yl)-amide

[(I_{eb}) ; A = 3-isoquinolyl ; B = -NR₂R₃ ; R₂ = R₄ = CH₃-CO ; R₃ = H]

Dans un ballon de 50 ml sous atmosphère d'azote on dissout 0,45 g (1,0 mmol) de (3R)isoquinoline-3-acide carboxylique (9-amino-4-oxo-1-phényl-3,4,6,7-tétrahydro-[1,4]diazépino[6,7,1-hi]indol-3-yl)-amide (produit de l'exemple 18) dans 10,0 ml de pyridine anhydre. On ajoute 3,0 ml (30 mmol) d'anhydride acétique, agite le mélange à 20-25 °C durant 30 minutes, puis précipite le milieu dans 100 ml d'eau glacée.

La solution est extraite par le dichlorométhane, la phase organique lavée à l'eau, desséchée, puis les solvants éliminés par distillation sous vide. Le résidu est repris par 100 ml d'éther éthylique et agité à 10 °C durant 30 minutes, le précipité est filtré et séché. Poids : 0,40 g. Rdt = 82 % - Poudre blanche - F = 280 °C
Analyse conforme pour C₂₉H₂₃N₅O₃ - C.C.M. : S.A; 0,20
R.M.N.¹Hδ(ppm) : 2,10 (s,3H); 3,00-3,10(m,1H); 3,20-3,30 (m,1H); 3,85-3,95 (m,1H); 4,50-4,60 (m,1H); 5,50 (d,1H); 7,10 (s,1H); 7,20-7,40 (m,4H); 7,50 (d,1H); 7,70-7,80 (m,2H); 7,95 (s,1H); 8,00 (d,1H); 8,10 (d,1H); 8,60 (s,1H); 8,75 (s, 1H); 9,30 (s,1H); 9,8 (d,1H)
LR. : 3300, 1650, 1490, 1380, 1250, 1160, 1050, 980, 860, 770, 750, 695 cm⁻¹

### Exemple 30 : (3R)N-(9-amino-4-oxo-1-phényl-3,4,6,7-tétrahydro-[1,4]diazépino[6,7,1-hi]indol-3-yl)-2-benzofuranecarboxamide

[(Iₑ) ; A = 2-benzofuranyl ; B = -NR₂R₃ ; R₂ = R₃ = H]

Préparation selon le mode opératoire de l'exemple 10 avec l'amine intermédiaire 2 et l'acide benzofurane-2-carboxylique. Après purification par chromatographie en éluant par le dichlorométhane progressivement enrichi en acétone, puis évaporation des solvants le produit est obtenu sous forme d'une poudre amorphe.
- Rdt = 22 % - Poudre jaune pâle - F = > 260 °C -

Analyse conforme pour C₂₆H₂₀N₄O₃ (0.25 H₂O) - C.C.M. : S.A10; 0,60
R.M.N.¹Hδ(ppm) : 3,00-3,15 (m,1H); 3,20-3,40 (m,1H); 3,50-4,00(m,2H); 3,95 (q,1H); 4,65 (t,1H); 5,65 (d,1H); 6,50 (s,1H); 6,85 (s,1H); 7,30 (t,1H); 7,35-7,40 (m,2H); 7,42-7,50 (m,2H); 7,55 (s,1H); 7,60 (d,3H); 7,70 (d,1H); 8,45 (d,1H)
I.R. : 3200, 1650, 1590, 1470, 1440, 1370, 1270, 1170, 840, 750, 690 cm⁻¹

### Exemple 31 : (3R)N-[4-oxo-1-phényl-9-(pyrrolidin-1-yl)-3,4,6,7-tétrahydro-[1,4]diazépino[6,7,1-hi]indol-3-yl]-isonicotinamide

[(Iₑₑ) ; A = 4-pyridyl ; B = -NR₂R₃ ; R₂ - R₃ = -(CH₂)₄-]

Dans un réacteur protégé de l'humidité on dissout dans 24,0 ml de pyridine anhydre 1,20g (3,46 mmol) de(3R)-3-amino-1-phényl-9-(pyrrolidin-1-yl)6,7-dihydro-3H-[1,4]diazépino [6,7,1-hi]indol-4-one (IIf) intermédiaire 4. On ajoute à la solution à une température < à 0°C, 0,92 g (5,17 mmol) du chlorhydrate du chlorure d'acide isonicotinique. La suspension est maintenue 48 heures sous agitation à 20 - 25°C sous atmosphère d'azote.

On ajoute ensuite 10 ml d'eau et 10 ml de dichlorométhane. La phase organique est séparée, la phase aqueuse extraite à nouveau par le dichlorométhane. Les phases organiques sont réunies, lavées par de l'eau puis séchées. Après évaporation des solvants le résidu est purifié par chromatographie rapide sur colonne de silice. L'élution par le dichlorométhane progressivement enrichi en méthanol permet d'obtenir, après élimination des solvants, le produit à l'état de pureté sous forme de poudre amorphe jaune.
Poids : 0,30 g - Rdt = 19,2% - F = 273 °C - C.C.M. : S.A10; 0,50
R.M.N,¹Hδ(ppm) : 2,00 (m,4H); 3,00 (m,1H); 3,20 (m,4H); 3,30 (m,1H); 3,90 (q,1H); 4,55 (q,1H); 5,50 (d,1H); 6,25 (s,1H); 6,70 (s,1H); 7,20-7,50 (m,3H); 7,60 (t,2H); 7,70 (d,2H); 8,00 (d,2H); 8,75 (d,2H).
I.R. : 3040, 1640, 1480, 1380, 1240, 1160, 1060, 1020, 900, 880, 840, 700, 600 cm⁻¹

### Exemple 32 : (3R)4,7-diméthyl-pyrazolo[5,1-c][1,2,4]triazine-3-carboxylique acide [4-oxo-1-phényl-9-(pyrrolidin-1-yl)-3,4,6,7-tétrahydro-[1,4]diazépino[6,7,1-hi]indol-3-yl]-amide

[(Iₑₑ);A. = 4,7-diméthyl-pyrazolo[5,1-c][1,2,4]-3-triazinyl; B =-NR₂R₃;R₂-R₃ = (CH₂)₄-]

Composé préparé selon le mode opératoire de l'exemple 2 et à partir de l'intermédiaire 4 de l'acide 4,7-diméthyl-pyrazolo[5,1-*c*[1,2,4]triazine-3-carboxylique. Le produit est purifié par chromatographie rapide sur silice en éluant par le dichlorométhane progressivement enrichi en méthanol.
Rdt = 20 % - Poudre amorphe jaune - F = > 300°C - C.C.M. : S.A8; 0,20
R.M.N.¹Hδ(ppm): 2,10 (m,4H); 2,60 (s,3H); 3,00 (m,1H); 3,15 (s,4H); 3,25 (s,3H); 3,30 (s,1H); 3,90 (q,1H); 4,60 (m,1H); 5,60 (d,1H); 6,25 (s,1H); 6,70 (s,1H); 7,00 (s,2H); 7,35 (m,3H); 7,60 (d,2H); 9,80 (d,1H).
I.R. : 1660, 1560, 1450, 1360, 1240, 700 cm⁻¹

### Partie biologique

### - Activité inhibitice de phosphodiestérase

La capacité des composés de formule (I) de l'invention à inhiber les phosphodiestérases des nucléotides cycliques est évaluée par la mesure de leur CI₅₀ (concentration nécessaire pour inhiber 50 % de l'activité enzymatique). Dans le cas des PDE4, cette valeur est comparée à la CI₅₀ du rolipram, inhibiteur spécifique de PDE4, par le rapport CI₅₀ du rolipram sur CI₅₀ du produit à tester vis-à-vis de la même préparation enzymatique.

Les différents types de phosphodiestérases sont obtenus partiellement purifiés sur colonne de DEAE-cellulose à partir de trachée de cobaye et d'aorte de chien selon une méthode adaptée de W.J. Thompson et al., 1979, Advances in Cyclic Nucleotide Research, Vol. 10 : 69-92, ed. G. Brooker et al. Raven Press, New York, et de P.J. Silver et al., 1988, Eur. J. Pharmacol. 150: 85-94, et à partir de la lignée cellulaire d'origine humaine U937, selon une méthode adaptée de T.J. Torphy *et al*., 1992, J. Pharm. Exp. Ther. 263 : 1195 - 1205.

Puis la mesure de l'activité enzymatique des différents types de PDE, et en particulier des PDE4, est faite selon une méthode également adaptée de W.J. Thompson, *Ibidem.* Pour la détermination de la CI₅₀, l'activité enzymatique est mesurée en présence de l'inhibiteur dans une gamme de concentrations de 0,1 à 100µM.

Le tableau suivant illustre l'activité inhibitrice de PDE4 comparativement à celle du rolipram sur une préparation d'enzyme obtenue à partir de la lignée U937.

| Ex. | CI₅₀ rolipram CI₅₀ exemple | Ex. | CI₅₀ rolipram CI₅₀ exemple |
|---|---|---|---|
| 1 | 1,9 | 18 | 11,0 |
| 4 | 33,0 | 19 | 1,4 |
| 8 | 3,4 | 20 | 1,1 |
| 12A | 2,0 | 21 | 3,3 |
| 12A (sel) | 5,7 | 30 | 15,0 |
| 13 | 1,6 | 32 | 4,0 |

L'examen des résultats du tableau précédent montre que les produits de l'invention testés dans l'essai inhibent généralement l'enzyme PDE4 d'origine humaine plus efficacement que le rolipram, et dans certains cas sont environ 30 fois plus actifs que le rolipram.

Par ailleurs des essais réalisés sur des PDE de différents types, purifiées à partir de trachée de cobaye ou d'aorte de chien, montrent que les valeurs de CI₅₀ obtenues avec les produits de l'invention vis-à-vis des PDE de type III et de type I et V sont beaucoup plus élevées que celles mesurées pour les PDE de type IV.

Ces résultats sont probants d'une activité inhibitrice puissante et sélective, pour les produits de l'invention, sur les PDE4.

### - Activité anti-inflammatoire et anti-allergique in vivo

Les effets des produits de l'invention ont été étudiés chez le cobaye dans un modèle d'infiltration d'éosinophiles induite par une stimulation antigénique ou par l'exposition à un aérosol de PAF selon une méthodologie décrite par Lagente V. *et al*., (1994) Br. J. Pharmacol. 112, 83P.

L'administration de produits des Exemples (1 - 30 mg/kg p.o.) diminue significativement le nombre d'éosinophiles dans le liquide de lange broncho-alvéolaire.

L'administration de produits de l'invention diminue également les réponses inflammatoires induites par l'instillation intatachéate d'IL-5 chez le cobaye.

Ces résultats démontrent l'activité anti-inflammatoire et/ou immuno-suppressive des produits de l'invention. Les produits de l'invention seront donc particulièrement utiles pour le traitement ou la prévention :
- des pathologies allergiques, et notamment l'asthme, la dermatite atopique ;
- des pathologies inflammatoires notamment au niveau de la bronche, mais aussi la polyarthrite rhumatoïde, et également les affections intestinales inflammatoires (rectocolite hémorragique et maladie de Crohn) ;
y compris lorsqu'il existe une composante auto-immune.

### Partie galénique

Les produits de l'invention sont administrés sous forme de compositions appropriées à la nature et à l'importance de l'affection à traiter. La posologie journalière chez l'homme est habituellement comprise entre 2 mg et 1 g de produit qui peut être absorbé en une ou plusieurs prises. Les compositions sont préparées sous des formes compatibles avec la voie d'administration envisagée, comme par exemple les comprimés, dragées, capsules, collutoires, aérosols, poudres pour inhalation, suppositoires, gels ou suspensions. Ces compositions sont préparées par des méthodes courantes pour l'homme de l'art et comprennent de 0,5 à 60 % en poids de principe actif (composé de formule I) et 40 à 99,5 % en poids de véhicule pharmaceutique approprié et compatible avec le principe actif et la forme physique de la composition envisagée. A titre d'exemple, on présente la composition et la préparation de comprimés contenant un composé de l'invention :

| | |
|---|---|
| Substance active de formule (I) | 1 à 75 mg |
| Lactose | 124 à 74 m g |
| Cellulose microcristalline | 36 à 60 mg |
| Polyvinylpyrrolidone | 6 mg |
| Carboxyméthylamidon sodique | 8 mg |
| Stéarate de magnésium | 1 mg |

Mélanger la substance active, le lactose, la cellulose microcristalline et le carboxyméthylamidon. Mouiller et granuler à l'aide d'une solution aqueuse ou alcoolique de polyvinylpyrrolidone de concentration adaptée. Sécher et calibrer le granulé. Mélanger de façon homogène le stéarate de magnésium.Comprimer à raison de 200 mg par comprimé.

## Revendications

1. Diazépino-indoles de formule (I) 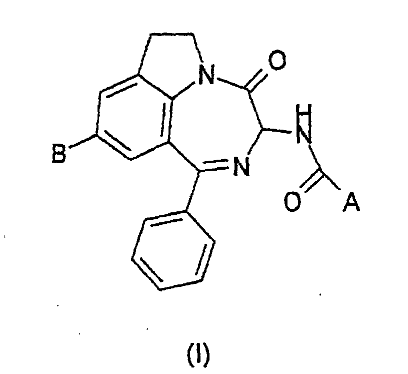 dans laquelle:
- A est aryle, hétéroaryle azoté, chacun éventuellement substitué par un à trois groupes indépendamment choisis parmi halogène, alkyle inférieur, haloalkyle inférieur, alkoxy inférieur, cycloalkyloxy, amino, alkyloxycarbonylamino inférieur ou alkylcarbonylamino inférieur;
- B est:
1°) -OR₁, R₁ étant -H ou R₄,
2°) -NR₂R₃, R₂ étant -C(NH)NH₂, et R₃ étant -H,
3°) -NR₂R₃, R₂ étant R₄, et R₃ étant -H,
4°) -NR₂R₃, R₂ et R₃ étant indépendamment -H ou alkyle inférieur, ou
5°) -N-R₂-R₃, R₂ et R₃ formant, ensemble avec l'atome azote auquel ils sont reliés, un
hétérocycle saturé de cinq à sept chaînons pouvant comprendre, comme second hétéroatome non relié directement à l'atome d'azote, un oxygène, un soufre, ou un azote;
- R₄ est :
1°) -CH₂-CO₂H,
2°) -CO-(CH₂)ₚ-CO₂H,
3°) -CO-A, où A est à la définition indiquée ci-dessus,
4°) -CO-CH = CH-CO₂H,
5°) -CO-(CH₂)ₙ-CH₃, n étant un entier égal ou supérieur à 0 et inférieur ou égal à 18,
6°) -CO-(CH₂-O-CH₂)ₚ-CH₂-O-CH₃,
7°) -CO-(CH₂-O-CH₂)ₚ-CO₂H,
8°) -(CH₂)ₚ-NR₅R₆, R₅ et R₆ étant indépendamment -H ou alkyle inférieur, ou
9°) -(CH₂)ₚ-N-R₅-R₆, R₅ et R₆ formant, ensemble avec l'atome d'azote auquel ils sont reliés, un hétérocycle saturé de cinq à sept chaînons pouvant comprendre, comme second hétéroatome non relié directement à l'atome d'azote, un oxygène, un soufre, ou un azote ;
- p est un entier égal à 2, 3, ou 4 ;
- étant entendu qu'inférieur, dans un radical comprenant une séquence alkyle, signifie que l'alkyle est linéaire ou ramifié et comporte de 1 à 4 atomes de carbone, ou est le cyclopropylméthyle.
leurs formes racémiques et leurs isomères de configuration déterminée notamment par le carbone 3 du noyau diazépino-indol-4-one,
ainsi que leurs sels pharmaceutiquement acceptables.

2. Diazépino-indoles de formule (I) selon la revendication 1 **caractérisés en ce que** leur configuration absolue est R selon la règle de Cahn-Ingold-Prelog, considéré l'atome de carbone asymétrique en position alpha par rapport au carbonyle du cycle diazépine,

3. Diazépino-indoles de formule (I) selon les revendications 1 et 2 **caractérisés en ce que** B est OR₁ ou NR₂R₃ où R₁R₂R₃ sont hydrogènes,

4. Diazépino-indoles de formule (I) selon l'une des revendications 1 à 3 dans laquelle A est aryle substitué par un à 3 groupes indépendamment choisi parmi halogène, amino, alkoxy inférieur.

5. Diazépino-indoles de formule (I) selon l'une des revendications 1 à 3 dans laquelle A est hétéroaryle azoté monocyclique comprenant de 1 à 2 atomes d'azote ou bicyclique comprenant de 1 à 4 atomes d'azote,

6. Diazépino-indoles de formule (I) selon la revendication 5 dans laquelle A est hétéroaryle substitué par des groupements amino, alkyles inférieurs, alkylcarbonylamino ou alkyloxycarbonylamino inférieurs.

7. Composés (I) selon les revendications 1 à 3 **caractérisés en ce qu'**ils sont :
- le (3R)Isoquinoline-3-acide carboxylique (9-hydroxy-4-oxo-1-phényl-3,4,6,7-tétrahydro-[1,4]diazépino[6,7,1-hi]indol-3-yl)-amide
- le (3R)4-t-butyloxycarbonylamino-N-(9-amino-4-oxo-1-phényl-3,4,6,7-tétrahydro-[ 1,4]diazépino[6,7,1-hi]indol-3-yl)-benzamide
- le (3R)4-amino-N-(9-amino-4-oxo-1-phényl-3,4,6,7-tétrahydro-[1,4]diazépino[6,7,1-hi]indol-3 -yl)-3,5-dichloro-benzamide
- le (3R)4-amino-N-(9-amine-4-oxo-1-phényl-3,4,6,7-tétrahydro-[1,4]diazépino[6,7,1-hi]indol-3-yl)-5-chloro-2-méthoxy-benzamide
- le (3R)N-(9-amino-4-oxo-1-phényl-3,4,6,7-tétrahydro-[1,4]diazépino[6,7,1-hi]indol-3-yl)-isonicotinamide
- le (3R)3-t.butyloxycarbonylamino-N-(9-amino-4-oxo-1-phényl-3,4,6,7-tétrahydro-[1,4]diazépino[6,7,1-hi]indol-3-yl)-isonicotinamide et son sel d'addition avec l'acide sulfurique
- le (3R)isoquinoline-3-acide carboxylique (9-amino-4-oxo-1-phényl-3,4,6,7-tétrahydro-[1,4]diazépino[6,7,1-hi]indol-3-yl)-amide
- le (3R)quinoline-3-acide carboxylique (9-amino-4-oxo-1-phényl-3,4,6,7-tétrahydro-[1,4]diazépino[6,7,1-hi]indol-3-yl)-amide
- le (3R)4,7-diméthyl-pyrazolo[5,1-c][1,2,4]triazine-3-acide carboxylique (9-amino-4-oxo-1-phényl-3,4,6,7-tétrahydro-[1,4]diazépino[6,7,1-hi]indol-3-yl)-amide
- le (3R)4-amino-3,5-dichloro-N-(9-diméthylamino-4-oxo-1-phényl-3,4,6,7-tétrahydro-[1,4]diazépino[6,7,1-hi]indol-3-yl)-benzamide
- le (3R)N-(9-amino-4-oxo-1-phényl-3,4,6,7-tétrahydro-[1,4]diazépino[6,7,1-hi]indol-3-yl)-2-benzofuranecarboxamide,
- le (3R)4,7-diméthyl-pyrazolo[5,1-c][1,2,4]triazine-3-carboxylique acide [4-oxo-1-phényl-9-(pyrrolidin-1-y)3,4,6,7-tétrahydro-[1,4]diazépino[6,7,1-hi]indol-3-yl]-amide,

8. Composé (I) selon les revendications 1 à 3 **caractérisé en ce qu'**il est le (3R)N-(9-amino-4-oxo-1-phényl-3,4,6,7-tétrahydro-[1,4]diazépino[6,7,1-hi]indol-3-yl)-nicotinamide

9. Amino-diazépino-indoles intermédiaires de préparation des composés de l'invention répondant à la formule (II) 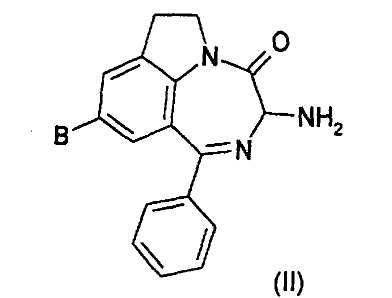 dans laquelle B est OR₁ ou NR₂R₃, R₁, R₂, R₃, étant hydrogènes,

10. Procédé de préparation des composés (I) 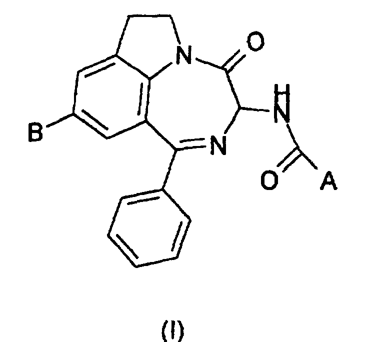 suivant la revendication 1, **caractérisé en ce qu'**il consiste :
**a**) pour obtenir les composés (I_{b}), de formule (I) dans laquelle B est un groupe -OH,
- à acyler un amino-diazépino-indole (II_{b}), de formule (II) telle que définie selon la revendication 9, dans laquelle B est un groupe -OH, par un réactif (III) de formule Z-CO-A, dans laquelle A est aryle, hétéroaryle azoté, chacun éventuellement substitué par un à trois groupes indépendamment choisis parmi halogène, alkyle inférieur, haloalkyle inférieur, alkoxy inférieur, cycloalkyloxy, amino, alkyloxycarbonylamino inférieur ou alkylcarbonylamino inférieur,
et Z représente un halogène, un groupe hydroxy, un groupe azido, un groupe imidazol-1-yle, un groupe -O-CO-Z₁, Z₁ pouvant être, outre A, un radical alkyle ou alkoxy encombré comportant de 3 à 6 atomes de carbone, ou encore Z peut être un groupe O-Z₂, Z₂ étant un groupe aromatique comportant un ou deux cycles substitués par un ou plusieurs radicaux nitro ou halogènes, ou
- à O- déméthyler en position 9 du noyau diazépino-indole un composé intermédiaire (I'_{c}) de formule 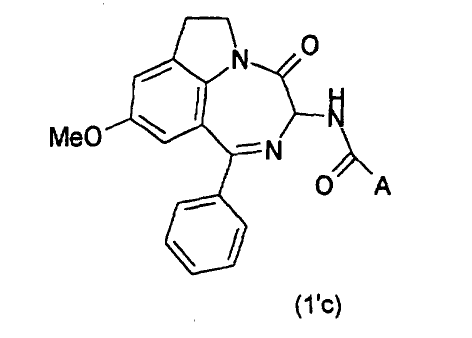 par un halogénure de bore ou d'aluminium, ou
- à diazoter dans un premier temps un composé (Iₑ), de formule (I) dans laquelle B est un groupe -NH₂, puis à hydrolyser dans un second temps le sel de diazonium intermédiaire, et
**b)** pour obtenir les composés (Iₑ) de l'invention, de formule (I) dans laquelle B est un groupe-NH₂,
- à acyler un composé (IIₑ), de formule (II) dans laquelle B est un groupe -NH₂, par le réactif (III) défini en a) de la présente revendication, ou
- à réduire le radical nitro d'un composé intermédiaire (I'_{d}) de formule 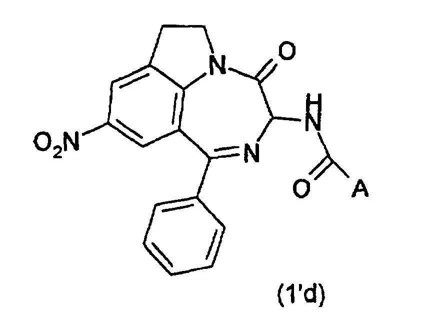 par l'action d'un métal tel que Zn ou Sn en milieu acide, ou celle d'un chlorure ou sulfure métallique tel que TiCl₃ ou Na₂S, et
**c)** pour obtenir les composés (I_{bb}) de l'invention, de formule (I) dans laquelle B est un groupe-O-CO-V ; V étant un groupe choisi parmi:
- A, tel que défini au a) de la présente revendication,
- (CH₂)ₚ-CO₂H, où p est un entier égal à 2, 3 ou 4,
- CH=CH-CO₂H,
- (CH₂)ₙ-CH₃, où n est un entier égal ou supérieur à 0 et inférieur ou égal à 18,
- (CH₂-O-CH₂)ₚ-CH₂-O-CH₃, où p est un entier égal à 2, 3 ou 4, ou
- (CH₂-O-CH₂)ₚ-CO₂H, où p est un entier égal à 2, 3 ou 4,
à estérifier un composé (I_{b}), défini en a), par un réactif (III') de formule V-CO-Z, dans laquelle Z a la signification définie en a) de la présente revendication, et
**d)** pour obtenir les composés (I_{bc}) de l'invention, de formule (I) dans laquelle B est un groupe-O-R₄, R₄ étant un groupe choisi parmi:
- CH₂-CO₂H,
- (CH₂)ₚ-NR₅R₆, où R₅ et R₆ sont indépendamment -H ou alkyle inférieur, ou
- (CH₂)ₚ-N-R₅-R₆, où R₅ et R₆ forment ensemble avec l'atome d'azote auquel ils sont reliés un hétérocycle,
à faire réagir un composé (I_{b}), défini en a), avec une base forte telle qu'un hydrure de métal alcalin pour former un phénate, lequel est mis à réagir avec un halogénure XR₄, et
**e)** pour obtenir les composés (Iₑₐ) de l'invention, de formule (I) dans laquelle B est un groupe-NH-C(NH)-NH₂,
à faire réagir un composé (Iₑ), défini en b), avec un agent de guanylation tel que le cyanamide, et
**f)** pour obtenir les composés (I_{eb}) de l'invention, de formule (I) dans laquelle B est un groupe-NH-CO-V, V ayant la signification définie en c) de la présente revendication,
à amidifier un composé (Iₑ), défini en b), par le réactif (III'): V-CO-Z défini en c) de la présente revendication, et
**g)** pour obtenir les composés (I_{ec}) de l'invention, de formule (I) dans laquelle B est un groupe-NH-R₂, R₂ étant alkyle inférieur ou un groupe R₄ tel que défini en d) de la présente revendication, à faire réagir un composé (Iₑ), défini en b), en présence d'une base forte avec un halogénure d'alkyle XR₂, et
**h)** pour obtenir les composés (I_{ed}) de l'invention, de formule (I) dans laquelle B est un groupe-NR₂R₃, R₂ et R₃ étant alkyles inférieurs,
à pratiquer l'alkylation réductive d'un composé (I_{ec}), défini en g) de la présente revendication, par un aldéhyde R'₃CHO dans lequel R'₃ est l'homologue immédiatement inférieur de R₃, et
**i)** pour obtenir les composés (Iee) de l'invention, de formule (I) dans laquelle B est un groupe-N-R₂-R₃, R₂ et R₃ formant un hétérocycle,
à effectuer une cyclisation par réaction d'un composé (Iₑ), défini en b), sur un réactif de formule
X-(CH₂)ₗ-Q-(CH₂)ₘ-X'
dans laquelle X et X', semblables ou différents, sont halogènes, Q est :
- une liaison simple de valence, et l et m sont des entiers allant de 1 à 3 avec l + m supérieur ou égal à 4 et inférieur ou égal à 6,
- un oxygène, un soufre ou un groupe -NH-, auquel cas l et m sont des entiers allant de 1 à 3 avec l + m supérieur ou égal à 3 et inférieur ou égal à 5, ou
à alkyler une amino diazépino-indole intermédiaire (IIf) de formule (II) dans laquelle B est un groupe -N-R₂-R₃, R₂ et R₃ formant avec l'atome d'azote un hétérocycle, par un réactif (III) de formule Z-CO-A tel que précédemment défini.

11. Utilisation d'un diazépino-indole selon l'une des revendications 1 à 8 pour préparer un médicament permettant de traiter des affections relevant d'une thérapie par un inhibiteur de phosphodiestérases 4.

12. Utilisation d'un diazépino-indole selon la revendication 11, **caractérisée en ce que** le médicament permet de prévenir ou de traiter des pathologies inflammatoires telles que l'asthme ou la polyarthrite rhumatoïde.

13. Médicament **caractérisé en ce qu'**il comprend un diazépino-indole tel qu'ils sont définis dans l'une des revendications 1 à 8.

## Claims

1. Diazepinoindoles of formula (I) 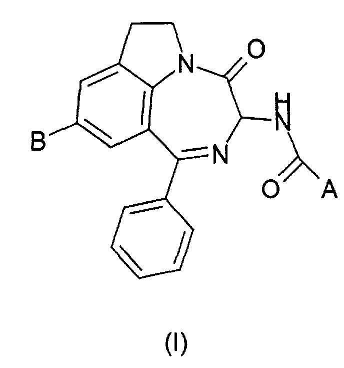 in which:
- A is aryl or nitrogen-containing heteroaryl, each optionally substituted by one to three groups selected independently from halogen, lower alkyl, lower haloalkyl, lower alkoxy, cycloalkyloxy, amino, lower alkyloxycarbonylamino or lower alkylcarbonylamino;
- B is:
1) -OR₁, where R₁ is -H or R₄,
2) -NR₂R₃, where R₂ is -C(NH)NH₂ and R₃ is -H,
3) -NR₂R₃, where R₂ is R₄ and R₃ is -H,
4) -NR₂R₃, where R₂ and R₃ are independently -H or lower alkyl, or
5) -N-R₂-R₃, where R₂ and R₃ form, together with the nitrogen atom to which they are attached, a saturated five- to seven-membered heterocycle which can comprise, as second heteroatom not attached directly to the nitrogen atom, an oxygen, a sulphur or a nitrogen;
- R₄ is:
1) -CH₂-CO₂H,
2) -CO-(CH₂)ₚ -CO₂H,
3) -CO-A, where A is as defined above,
4) -CO-CH = CH-CO₂H,
5) -CO-(CH₂)ₙ-CH₃, where n is an integer equal to or greater than 0 and less than or equal to 18,
6) -CO-(CH₂-O-CH₂)ₚ-CH₂-O-CH₃,
7) -CO-(CH₂-O-CH₂)ₚ-CO₂H,
8) -(CH₂)ₚ-NR₅R₆, where R₅ and R₆ are independently -H or lower alkyl, or
9) -(CH₂)ₚ-N-R₅-R₆, where R₅ and R₆ form, together with the nitrogen atom to which they are attached, a saturated five- to seven-membered heterocycle which can comprise, as second heteroatom not attached directly to the nitrogen atom, an oxygen, a sulphur or a nitrogen;
- p is an integer equal to 2, 3 or 4;
- where lower, in a radical comprising an alkyl chain, is understood to mean that the alkyl is linear or branched and contains from 1 to 4 carbon atoms, or is cyclopropylmethyl;
racemic forms and isomers thereof with a configuration in particular determined by carbon 3 of the diazepinoindol-4-one ring, and pharmaceutically acceptable salts thereof.

2. Diazepinoindoles of formula (I) according to Claim 1, **characterized in that** their absolute configuration is R according to the Cahn-Ingold-Prelog rule, considering the asymmetric carbon atom in the position alpha to the carbonyl of the diazepine ring.

3. Diazepinoindoles of formula (I) according to Claims 1 and 2, **characterized in that** B is OR₁ or NR₂R₃ where R₁R₂R₃ are hydrogen.

4. Diazepinoindoles of formula (I) according to one of Claims 1 to 3, in which A is aryl substituted by one to 3 groups independently selected from halogen, amino and lower alkoxy.

5. Diazepinoindoles of formula (I) according to one of Claims 1 to 3, in which A is monocyclic nitrogen-containing heteroaryl comprising from 1 to 2 nitrogen atoms or bicyclic nitrogen-containing heteroaryl comprising from 1 to 4 nitrogen atoms.

6. Diazepinoindoles of formula (I) according to Claim 5, in which A is heteroaryl substituted by amino, lower alkyl, lower alkylcarbonylamino or lower alkyloxycarbonylamino groups.

7. Compounds (I) according to Claims 1 to 3, **characterized in that** they are:
- isoquinoline-3-carboxylic acid (3R)-(9-hydroxy-4-oxo-1-phenyl-3,4,6,7-tetrahydro-[1,4]diazepino[6,7,1-hi]indol-3-yl)amide
- (3R)-4-t-butyloxycarbonylamino-N-(9-amino-4-oxo-1-phenyl-3,4,6,7-tetrahydro-[1,4] diazepino[6,7,1-hi]indol-3-yl)benzamide
- (3R)-4-amino-N-(9-amino-4-oxo-1-phenyl-3,4,6,7-tetrahydro[1,4]diazepino[6,7,1-hi]indol-3-yl)-3,5-dichlorobenzamide
- (3R)-4-amino-N-(9-amino-4-oxo- 1-phenyl-3,4,6,7-tetrahydro[1,4]diazepino[6,7,1-hi]indol-3-yl)-5-chloro-2-methoxybenzamide
- (3R)-N-(9-amino-4-oxo-1-phenyl-3,4,6,7-tetrahydro[1,4]diazepino[6,7,1-hi]indol-3-yl)iso-nicotinamide
- (3R)-3-t-butyloxycarbonylamino-N-(9-amino-4-oxo-1-phenyl-3,4,6,7-tetrahydro-[1,4] diazepino[6,7,1-hi]indol-3-yl)isonicotinamide and its addition salt with sulphuric acid
- isoquinoline-3-carboxylic acid (3R)-(9-amino-4-oxo-1-phenyl-3,4,6,7-tetrahydro-[1,4]-diazepino[6,7,1-hi]indol-3-yl)amide
- quiholine-3-carboxylic acid (3R)-(9-amino-4-oxo-1-phenyl-3,4,6,7-tetrahydro-[1,4]diazepino-[6,7,1-hi]indol-3-yl)amide
- 4,7-dimethylpyrazolo[5,1-c][1,2,4]triazine-3-carboxylic acid (3R)-(9-amino-4-oxo-1-phenyl-3,4,6,7-tetrahydro[1,4]diazepino[6,7,1-hi]indol-3-yl)amide
- (3R)-4-amino-3,5-dichloro-N-(9-dimethylamino-4-oxo-1-phenyl-3,4,6,7-tetrahydro-[1,4]-diazepino[6,7,1-hi]indol-3-yl)benzamide
- (3R)-N-(9-amino-4-oxo-1-phenyl-3,4,6,7-tetrahydro[1,4]diazepino[6,7,1-hi]indol-3-yl)-2-benzofuranecarboxamide
- 4,7-dimethylpyrazolo[5,1-c][1,2,4]triazine-3-carboxylic acid (3R)-[4-oxo-1-phenyl-9-(pyrrolidin-1-yl)-3,4,6,7-tetrahydro[1,4]diazepino[6,7,1-hi]indol-3-yl]amide.

8. Compound (I) according to Claims 1 to 3, **characterized in that** it is (3R)-N-(9-amino-4-oxo-1-phenyl-3,4,6,7-tetrahydro[1,4]diazepino[6,7,1-hi]indol-3-yl)nicotinamide.

9. Aminodiazepinoindoles which are intermediates in the preparation of compounds of the invention, corresponding to the formula (II) 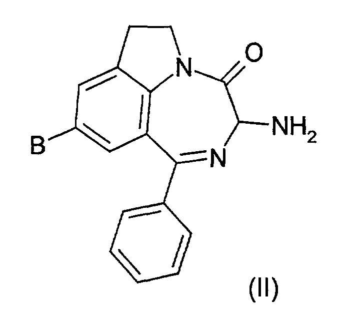 in which B is OR₁ or NR₂R₃, where R₁, R₂, R₃ are hydrogen.

10. Process for the preparation of the compounds (I) 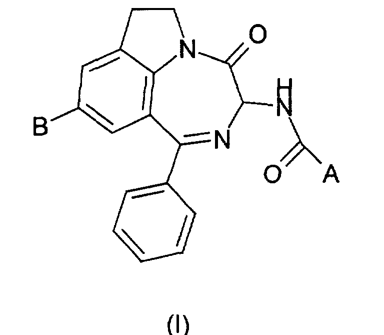 according to Claim 1, **characterized in that** it consists:
**a)** in order to obtain the compounds (I_{b}) of formula (I), in which B is an -OH group,
- in acylating an aminodiazepinoindole (II_{b}) of formula (II) as defined in Claim 9, in which B is an -OH group, with a reactant (III) of formula Z-CO-A, in which A is aryl or nitrogen-containing heteroaryl, each optionally substitued by one to three groups selected independently from halogen, lower alkyl, lower haloalkyl, lower alkoxy, cycloalkyloxy, amino, lower alkyloxycarbonylamino or lower alkylcarbonylamino,
and Z represents a halogen, a hydroxyl group, an azido group, an imidazol-1-yl group, or a group -O-CO-Z₁, where Z₁ may be, besides A, a bulky alkyl or alkoxy radical containing from 3 to 6 carbon atoms, or alternatively Z may be a group O-Z₂, where Z₂ is an aromatic group containing one or two rings substituted by one or more nitro or halogen radicals, or
- in O-demethylating, in position 9 of the diazepinoindole ring, an intermediate compound (I'_{c}) of formula 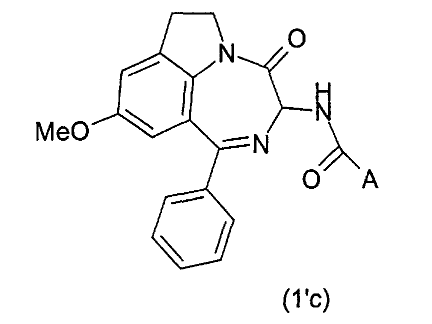 with a boron or aluminium halide, or
- in diazotizing, in a first step, a compound (Iₑ) of formula (I), in which B is an -NH₂ group, and then in hydrolysing, in a second step, the intermediate diazonium salt, and
**b)** in order to obtain the compounds (Iₑ) of the invention, of formula (I), in which B is an -NH₂ group,
- in acylating a compound (IIₑ) of formula (II), in which B is an -NH₂ group, with the reactant (III) defined in a) of this claim, or
- in reducing the nitro radical of an intermediate compound (I'_{d}) of formula 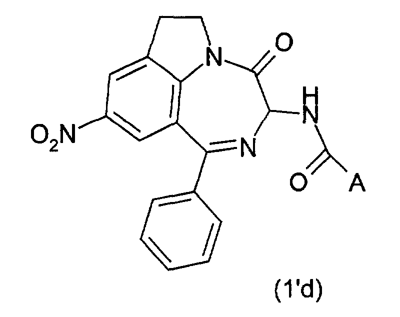 by the action of a metal such as Zn or Sn in an acidic medium, or by the action of a metal chloride or sulphide such as TiCl₃ or Na₂S, and
**c)** in order to obtain the compounds (I_{bb}) of the invention, of formula (I), in which B is a group
- O-CO-V; where V is a group selected from:
- A, as defined in a) of this claim,
- (CH₂)ₚ-CO₂H, where p is an integer equal to 2, 3 or 4,
- CH = CH-CO₂H,
- (CH₂)ₙ-CH₃, where n is an integer equal to or greater than 0 and less than or equal to 18,
- (CH₂-O-CH₂)ₚ-CH₂-O-CH₃, where p is an integer equal to 2, 3 or 4, or
- (CH₂-O-CH₂)ₚ-CO₂H, where p is an integer equal to 2, 3 or 4,
in esterifying a compound (I_{b}), defined in a), with a reactant (III') of formula V-CO-Z, in which Z is as defined in a) of this claim, and
**d)** in order to obtain the compounds (I_{bc}) of the invention, of formula (I), in which B is a group
- O-R₄, where R₄ is a group selected from:
- CH₂-CO₂H,
- (CH₂)ₚ-NR₅R₆, where R₅ and R₆ are independently -H or lower alkyl, or
- (CH₂)ₚ-N-R₅-R₆, where R₅ and R₆ form, together with the nitrogen atom to which they are attached, a heterocycle,
in reacting a compound (I_{b}), defined in a), with a strong base such as an alkali metal hydride, in order to form a phenate, which is reacted with a halide XR₄, and
**e)** in order to obtain the compounds (Iₑₐ) of the invention, of formula (I), in which B is a group -NH-C(NH)-NH₂,
in reacting a compound (Iₑ), defined in b), with a guanylating agent such as cyanamide, and
**f)** in order to obtain the compounds (I_{eb}) of the invention, of formula (I), in which B is a group -NH-CO-V, where V is as defined in c) of this claim,
in amidating a compound (Iₑ), defined in b), with the reactant (III'), where V-CO-Z is as defined in c) of this claim, and
**g)** in order to obtain the compounds (I_{ec}) of the invention, of formula (I), in which B is a group -NH-R₂, where R₂ is lower alkyl or a group R₄ as defined in d) of this claim,
in reacting a compound (Iₑ), defined in b), with an alkyl halide XR₂ in the presence of a strong base, and
**h)** in order to obtain the compounds (I_{ed}) of the invention, of formula (I), in which B is a group -NR₂R₃, where R₂ and R₃ are lower alkyls,
in reductively alkylating a compound (I_{ec}), defined in g) of this claim, with an aldehyde R'₃CHO in which R'₃ is the immediately lower homologue of R₃, and
**i)** in order to obtain the compounds (Iₑₑ) of the invention, of formula (I), in which B is a group -N-R₂-R₃, where R₂ and R₃ form a heterocycle,
in performing a cyclization by reaction of a compound (Iₑ), defined in b), with a reactant of formula
X-(CH₂)ₗ-Q-(CH₂)ₘ-X'
in which X and X', which may be the same or different, are halogens, Q is:
- a single valency bond, and l and m are integers ranging from 1 to 3 with l + m greater than or equal to 4 and less than or equal to 6,
- an oxygen, a sulphur or a group -NH-, in which case l and m are integers ranging from 1 to 3 with l + m greater than or equal to 3 and less than or equal to 5, or
in alkylating an aminodiazepinoindole intermediate (IIf) of formula (II), in which B is a group -N-R₂-R₃, where R₂ and R₃ form, together with the nitrogen atom, a heterocycle, with a reactant (III) of formula Z-CO-A as defined above.

11. Use of a diazepinoindole according to one of Claims 1 to 8 for preparing a medicament which makes it possible to treat complaints which are amenable to therapy by a phosphodiesterase 4 inhibitor.

12. Use of a diazepinoindole according to Claim 11, **characterized in that** the medicament makes it possible to prevent or treat inflammatory pathologies such as asthma or rheumatoid arthritis.

13. Medicament, **characterized in that** it comprises a diazepinoindole as defined in one of Claims 1 to 8.

## Patentansprüche

1. Diazepinoindole der Formel (1) 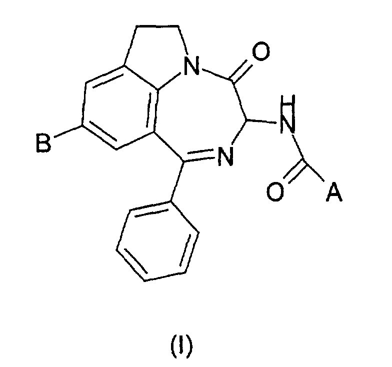 worin:
- A für jeweils gegebenenfalls durch eine bis drei unabhängig unter Halogen, Niederalkyl, Halogenniederalkyl, Niederalkoxy, Cycloalkyloxy, Amino, Niederalkyloxycarbonylamino oder Niederalkylcarbonylamino ausgewählte Gruppen substituiertes Aryl oder stickstoffhaltiges Heteroaryl steht;
- B für:
1) -OR₁, wobei R₁ -H oder R₄ bedeutet,
2) -NR₂R₃, wobei R₂ -C(NH)NH₂ und R₃ -H bedeutet,
3) -NR₂R₃, wobei R₂ R₄ und R₃ -H bedeutet,
4) -NR₂R₃, wobei R₂ und R₃ unabhängig voneinander -H oder Niederalkyl bedeuten, oder
5) -N-R₂-R₃, wobei R₂ und R₃ zusammen mit dem Stickstoffatom, an das sie gebunden sind, einen fünf- bis siebengliedrigen gesättigten Heterocyclus bilden, der als zweites nicht direkt an das Stickstoffatom gebundenes Heteroatom Sauerstoff, Schwefel oder Stickstoff enthalten kann, steht;
- R₄ für:
1) -CH₂-CO₂H,
2) -CO-(CH₂)ₚ -CO₂H,
3) -CO-A, worin A die oben angegebene Bedeutung hat,
4) -CO-CH = CH-CO₂H,
5) -CO-(CH₂)ₙ-CH₃, wobei n eine ganze Zahl größer gleich 0 und kleiner gleich 18 bedeutet,
6) -CO-(CH₂-O-CH₂)ₚ-CH₂-O-CH₃,
7) -CO-(CH₂-O-CH₂)ₚ-CO₂H,
8) -(CH₂)ₚ-NR₅R₆, worin R₅ und R₆ unabhängig voneinander -H oder Niederalkyl bedeuten, oder
9) -(CH₂)ₚ-N-R₅-R₆, wobei R₅ und R₆ zusammen mit dem Stickstoffatom, an das sie gebunden sind, einen fünf- bis siebengliedrigen gesättigten Heterocyclus bilden, der als zweites, nicht direkt an das Stickstoffatom gebundenes Heteroatom Sauerstoff, Schwefel oder Stickstoff enthalten kann, steht;
- p für die ganze Zahl 2, 3 oder 4 steht;
- wobei in einem Rest mit einer Alkylkette unter "nieder" zu verstehen ist, daß das Alkyl linear oder verzweigt ist und 1 bis 4 Kohlenstoffatome enthält, oder Cyclopropylmethyl ist;
racemische Formen und Isomere davon mit insbesondere durch das Kohlenstoffatom 3 des Diazepinoindol-4-on-Rings bestimmter Konfiguration sowie pharmazeutisch unbedenkliche Salze davon.

2. Diazepinoindole der Formel (I) nach Anspruch 1, **dadurch gekennzeichnet, daß** ihre absolute Konfiguration nach Cahn-Ingold-Prelog bei Betrachtung des asymmetrischen Kohlenstoffatoms in alpha-Position zur Carbonylgruppe des Diazepinrings R ist.

3. Diazepinoindole der Formel (I) nach Anspruch 1 und 2, **dadurch gekennzeichnet, daß** B für OR₁ oder NR₂R₃, worin R₁, R₂ und R₃ Wasserstoff bedeuten, steht.

4. Diazepinoindole der Formel (I) nach einem der Ansprüche 1 bis 3, worin A für durch eine bis 3 unabhängig unter Halogen, Amino und Niederalkoxy ausgewählte Gruppen substituiertes Aryl steht.

5. Diazepinoindole der Formel (I) nach einem der Ansprüche 1 bis 3, worin A für monocyclisches stickstoffhaltiges Heteroaryl mit 1 bis 2 Stickstoffatomen oder bicyclisches stickstoffhaltiges Heteroaryl mit 1 bis 4 Stickstoffatomen steht.

6. Diazepinoindole der Formel (I) nach Anspruch 5, worin A für durch Amino-, Niederalkyl-, Niederalkylcarbonylamino- oder Niederalkyloxycarbonylaminogruppen substituiertes Heteroaryl steht.

7. Verbindungen (I) nach den Ansprüchen 1 bis 3, **dadurch gekennzeichnet, daß** es sich dabei um:
- Isochinolin-3-carbonsäure-(3R)-(9-hydroxy-4-oxo-1-phenyl-3,4,6,7-tetrahydro-[1,4]diazepino-[6,7,1-hi]indol-3-yl)amid,
- (3R)-4-t-Butyloxycarbonylamino-N-(9-amino-4-oxo-1-phenyl-3,4,6,7-tetrahydro-[1,4]diazepino[6,7,1-hi]indol-3-yl)benzamid,
- (3R)-4-Amino-N-(9-amino-4-oxo-1-phenyl-3,4,6,7-tetrahydro-[1,4]diazepino[6,7,1-hi]indol-3-yl)-3,5-dichlorbenzamid,
- (3R)-4-Amino-N-(9-amino-4-oxo-1-phenyl-3,4,6,7-tetrahydro-[1,4]diazepino[6,7,1-hi]indol-3-yl)-5-chlor-2-methoxybenzamid,
- (3R)-N-(9-Amino-4-oxo-1-phenyl-3,4,6,7-tetrahydro-[1,4]diazepino[6,7,1-hi]indol-3-yl)-isonicotinamid,
- (3R)-3-t-Butyloxycarbonylamino-N-(9-amino-4-oxo-1-phenyl-3,4,6,7-tetrahydro-[1,4]diazepino[6,7,1-hi]indol-3-yl)isonicotinamid und dessen Schwefelsäure-Additionssalz,
- Isochinolin-3-carbonsäure-(3R)-(9-amino-4-oxo-1-phenyl-3,4,6,7-tetrahydro-[1,4]diazepino-[6,7,1-hi]indol-3-yl)amid,
- Chinolin-3-carbonsäure-(3R)-(9-amino-4-oxo-1-phenyl-3,4,6,7-tetrahydro-[1,4]diazepino-[6,7,1-hi]indol-3-yl)amid,
- 4,7-Dimethyl-pyrazolo[5,1-c][1,2,4]triazin-3-carbonsäure-(3R)-(9-amino-4-oxo-1-phenyl-3,4,6,7-tetrahydro-[1,4]diazepino[6,7,1-hi]indol-3-yl)amid,
- (3R)-4-Amino-3,5-dichlor-N-(9-dimethylamino-4-oxo-1-phenyl-3,4,6,7-tetrahydro-[ 1,4]diazepino[6,7,1-hi]indol-3-yl)benzamid,
- (3R)-N-(9-Amino-4-oxo-1-phenyl-3,4,6,7-tetrahydro-[1,4]diazepino[6,7,1-hi]indol-3-yl)-2-benzofurancarboxamid und
- 4,7-Dimethyl-pyrazolo[5,1-c][1,2,4]triazin-3-carbonsäure-(3R)-[4-oxo-1-phenyl-9-(pyrrolidin-1-yl)-3,4,6,7-tetrahydro-[1,4]diazepino[6,7,1-hi]indol-3-yl]amid
handelt.

8. Verbindung (I) nach den Ansprüchen 1 bis 3, **dadurch gekennzeichnet, daß** es sich dabei um (3R)-N-(9-Amino-4-oxo-1-phenyl-3,4,6,7-tetrahydro-[1,4]diazepino[6,7,1-hi]indol-3-yl)-nicotinamid
handelt.

9. Aminodiazepinoindole, die Zwischenprodukte bei der Herstellung der erfindungsgemäßen Verbindungen darstellen, mit der Formel (II) 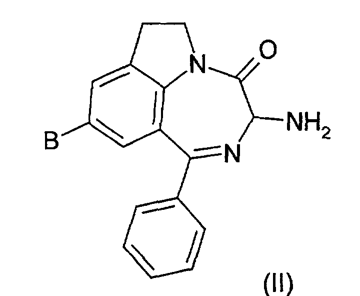 worin B für OR₁ oder NR₂R₃, wobei R₁, R₂ und R₃ Wasserstoff bedeuten, steht.

10. Verfahren zur Herstellung der Verbindungen (I) 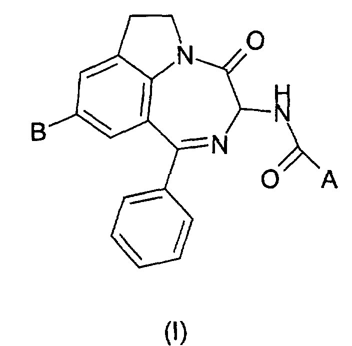 gemäß Anspruch 1, **dadurch gekennzeichnet, daß** man:
a) zur Herstellung von Verbindungen (I_{b}) der Formel (I), worin B für eine -OH-Gruppe steht,
- ein Aminodiazepinoindol (II_{b}) der Formel (II) gemäß Anspruch 9, worin B für eine -OH-Gruppe steht, mit einem Reagenz (III) der Formel Z-CO-A, worin A für jeweils gegebenenfalls durch eine bis drei unabhängig unter Halogen, Niederalkyl, Halogenniederalkyl, Niederalkoxy, Cycloalkyloxy, Amino, Niederalkyloxycarbonylamino oder Niederalkylcarbonylamino ausgewählte Gruppen substituiertes Aryl oder stickstoffhaltiges Heteroaryl steht;
und Z für ein Halogen, eine Hydroxylgruppe, eine Azidgruppe, eine Imidazol-1-yl-Gruppe, eine Gruppe -O-CO-Z₁, worin Z₁ neben A einen sperrigen Alkyl- oder Alkoxyrest mit 3 bis 6 Kohlenstoffatomen bedeuten kann, oder eine Gruppe O-Z₂, worin Z₂ eine aromatische Gruppe mit einem oder zwei, durch einen oder mehrere Nitro- oder Halogenreste substituierten Ringen bedeutet, steht, acyliert, oder
- den Diazepinoindolring einer Zwischenverbindung (I'_{c}) der Formel 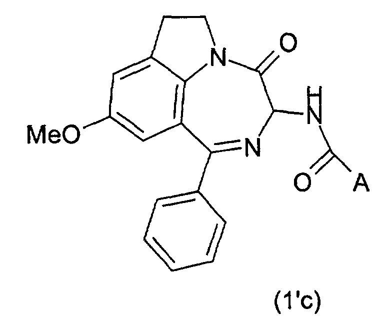 mit einem Bor- oder Aluminiumhalogenid in 9-Position O-entmethyliert, oder
- zunächst eine Verbindung (Iₑ) der Formel (I), worin B für eine -NH₂-Gruppe steht, diazotiert und danach das Diazoniumsalz-Zwischenprodukt hydrolysiert, und
**b)** zur Herstellung von erfindungsgemäßen Verbindungen (Iₑ) der Formel (I), worin B für eine -NH₂-Gruppe steht,
- eine Verbindung (IIₑ) der Formel (II), worin B für eine -NH₂-Gruppe steht, mit dem unter Punkt a) dieses Anspruchs definierten Reagenz (III) acyliert, oder
- den Nitrorest einer Zwischenverbindung (I'_{d}) der Formel 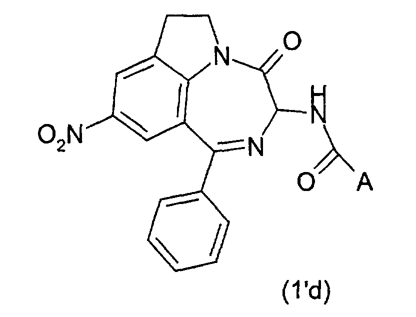 durch Einwirkung eines Metalls wie Zn oder Sn in saurem Milieu oder durch Einwirkung eines Metallchlorids oder -sulfids wie TiCl₃ oder Na₂S reduziert, und
**c)** zur Herstellung von erfindungsgemäßen Verbindungen (I_{bb}) der Formel (I), worin B für eine Gruppe -O-CO-V steht; wobei V eine unter:
- A in der unter Punkt a) dieses Anspruchs angegebenen Bedeutung,
- (CH₂)ₚ-CO₂H, worin p für die ganze Zahl 2, 3 oder 4 steht,
- CH = CH-CO₂H,
- (CH₂)ₙ-CH₃, worin n für eine ganze Zahl größer gleich 0 und kleiner gleich 18 steht,
- (CH₂-O-CH₂)ₚ-CH₂-O-CH₃, worin p für die ganze Zahl 2, 3 oder 4 steht, oder
- (CH₂-O-CH₂)ₚ-CO₂H, worin p für die ganze Zahl 2, 3 oder 4 steht,
ausgewählte Gruppe bedeutet,
eine Verbindung (I_{b}) gemäß der unter Punkt a) aufgeführten Definition mit einem Reagenz (III') der Formel V-CO-Z, worin Z die unter Punkt a) dieses Anspruchs angegebene Bedeutung hat, verestert, und
**d)** zur Herstellung von erfindungsgemäßen Verbindungen (I_{bc}) der Formel (I), worin B für eine Gruppe -O-R₄, steht, wobei R₄ eine unter:
- CH₂-CO₂H,
- (CH₂)ₚ-NR₅R₆, worin R₅ und R₆ unabhängig voneinander für -H oder Niederalkyl stehen, oder
- (CH₂)ₚ-N-R₅-R₆, worin R₅ und R₆ zusammen mit dem Stickstoffatom, an das sie gebunden sind, einen Heterocyclus bilden,
ausgewählte Gruppe bedeutet,
eine Verbindung (I_{b}) gemäß der unter Punkt a) aufgeführten Definition mit einer starken Base wie einem Alkalimetallhydrid zu einem Phenolat umsetzt, welches mit einem Halogenid XR₄ umgesetzt wird, und
**e)** zur Herstellung von erfindungsgemäßen Verbindungen (Iₑₐ) der Formel (I), worin B für eine Gruppe -NH-C(NH)-NH₂ steht,
eine Verbindung (Iₑ) gemäß der unter Punkt b) aufgeführten Definition mit einem Guanylierungsmittel wie Cyanamid umsetzt, und
**f)** zur Herstellung von erfindungsgemäßen Verbindungen (I_{eb}) der Formel (I), worin B für eine Gruppe -NH-CO-V steht, wobei V die unter Punkt c) dieses Anspruchs angegebene Bedeutung hat,
eine Verbindung (Iₑ) gemäß der unter Punkt b) aufgeführten Definition mit dem Reagenz (III') umsetzt, wobei V-CO-Z V die unter Punkt c) dieses Anspruchs angegebene Bedeutung hat, und
**g)** zur Herstellung von erfindungsgemäßen Verbindungen (I_{ec}) der Formel (I), worin B für eine Gruppe -NH-R₂, wobei R₂ Niederalkyl oder eine Gruppe R₄ gemäß der unter Punkt d) aufgeführten Definition bedeutet, steht,
eine Verbindung (Iₑ) gemäß der unter Punkt b) aufgeführten Definition in Gegenwart einer starken Base mit einem Alkylhalogenid XR₂ umsetzt, und
**h)** zur Herstellung von erfindungsgemäßen Verbindungen (I_{ed}) der Formel (I), worin B für eine Gruppe -NR₂R₃, worin R₂ und R₃ Niederalkyl bedeuten, steht,
eine Verbindung (I_{ec}), gemäß der unter Punkt g) dieses Anspruchs aufgeführten Definition mit einem Aldehyd R'₃CHO, worin R'₃ für das nächstniedrigere Homolog von R₃ steht, reduktiv alkyliert, und
**i)** zur Herstellung von erfindungsgemäßen Verbindungen (Iₑₑ) der Formel (I), worin B für eine Gruppe -N-R₂-R₃, worin R₂ und R₃ einen Heterocyclus bilden, steht,
durch Umsetzung einer Verbindung (Iₑ) gemäß der unter Punkt b) aufgeführten Definition mit einem Reagenz der Formel
X-(CH₂)ₗ-Q-(CH₂)ₘ-X'
worin X und X' gleich oder verschieden sind und für Halogen stehen, Q für:
- eine einfache Valenzbindung steht und 1 und m für ganze Zahlen von 1 bis 3 mit l + m größer gleich 4 und kleiner gleich 6 stehen,
- Sauerstoff, Schwefel oder eine -NH-Gruppe steht, wobei in diesem Fall 1 und m für ganze Zahlen von 1 bis 3 mit l + m größer gleich 3 und kleiner gleich 5 stehen,
eine Cyclisierung durchführt, oder
ein Aminodiazepinoindol-Zwischenprodukt (IIf) der Formel (II), worin B für eine Gruppe
-N-R₂-R₃, wobei R₂ und R₃ mit dem Stickstoffatom einen Heterocyclus bilden, steht, mit einem Reagenz (III) der Formel Z-CO-A gemäß obiger Definition alkyliert.

11. Verwendung eines Diazepinoindols nach einem der Ansprüche 1 bis 8 zur Herstellung eines Arzneimittels, das die Behandlung von Beschwerden ermöglicht, die einer Therapie mit einem Phosphodiesterase-4-Inhibitor zugänglich sind.

12. Verwendung eines Diazepinoindols nach Anspruch 11, **dadurch gekennzeichnet, daß** das Arzneimittel die Prävention oder Behandlung von Entzündungserkrankungen wie Asthma oder rheumatoider Arthritis ermöglicht.

13. Arzneimittel, **dadurch gekennzeichnet, daß** es ein Diazepinoindol nach einem der Ansprüche 1 bis 8 enthält.
